(19) Europäisches Patentamt
European Patent Office
Office européen des brevets

(11) EP 0 539 967 B1

(12) EUROPEAN PATENT SPECIFICATION

(45) Date of publication and mention of the grant of the patent:
**09.09.1998 Bulletin 1998/37**

(51) Int Cl.[6]: **G01N 21/64**

(21) Application number: **92118462.8**

(22) Date of filing: **29.10.1992**

(54) **Sensors and methods for sensing**

Sensoren und Bestimmungsverfahren

Capteurs et procédés d'analyse

(84) Designated Contracting States:
**AT BE DE DK ES FR GB IT NL SE**

(30) Priority: **31.10.1991 US 786014**

(43) Date of publication of application:
**05.05.1993 Bulletin 1993/18**

(73) Proprietor: **MINNESOTA MINING AND MANUFACTURING COMPANY**
**St. Paul, Minnesota 55133-3427 (US)**

(72) Inventors:
- **Nagel, Colleen C., c/o Minnesota Mining and St. Paul, Minnesota 55133-3427 (US)**
- **Bentsen, James G., c/o Minnesota Mining and St. Paul, Minnesota 55133-3427 (US)**

(74) Representative:
**Hilleringmann, Jochen, Dipl.-Ing. et al**
**Patentanwälte**
**von Kreisler-Selting-Werner,**
**Postfach 10 22 41**
**50462 Köln (DE)**

(56) References cited:

| | |
|---|---|
| EP-A- 263 693 | EP-A- 363 219 |
| US-A- 4 803 049 | US-A- 5 037 615 |

- **ANALYTICA CHEMICA ACTA vol. 212, 1988, pages 261 - 265 A. SHARMA AND O.S. WOLFBEIS 'Unusually efficient quenching of the fluorescence of an energy transfer-based optical sensor for oxygen'**
- **ANALYTICAL CHEMISTRY vol. 62, no. 22, 15 November 1990, COLUMBUS US pages 2461 - 2466 A. UENO ET AL. 'Host-guest sensory systems for detecting organic compounds'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 539 967 B1

## Description

The present invention relates to sensing or determining the concentration of an analyte of interest in a medium. More particularly, the invention relates to sensor apparatus or systems and methods for sensing the concentration of an analyte of interest, for example, oxygen, in a medium, for example, blood.

It is sometimes necessary or desirable for a physician to determine the concentration of certain gases, e.g., oxygen and carbon dioxide, in blood. This can be accomplished utilizing an optical sensor which contains an optical indicator responsive to the component or analyte of interest. The optical sensor is exposed to the blood, and excitation light is provided to the sensor so that the optical indicator can provide an optical signal indicative of a characteristic of the analyte of interest. For example, the optical indicator may fluoresce and provide a fluorescent optical signal as described in U.S.-A-RE31,897 or it may function on the principles of light absorbance as described, for example, in U.S.-A-4,041,932.

The use of optical fibers has been suggested as part of such sensor systems. The optical indicator is placed at the end of an elongated optical fiber which is placed in the medium to be analyzed. This approach has many advantages, particularly when it is desired to determine a concentration of analyte in a medium inside a patient's body. The optical fiber/indicator combination can be made sufficiently small in size to easily enter and remain in the cardiovascular system of the patient. Consistent and accurate concentration determinations are obtained.

Luminescence measurement analysis for monitoring concentrations of analytes is well known in the art. Generally, a calibration curve of light intensity (or a function of intensity) vs. concentration of the analyte is made. This method may involve a determination of absolute light intensities of both excitation and emission. When luminescent indicators are excited by an intensity modulated excitation source, the phase shift between the excitation and emission signals can be used to determine an analyte dependent luminescence lifetime.

One problem which may exist in such systems is the wavelength proximity between the excitation signal (light) and the emission signal (light) of the indicator. In many cases, the excitation signal and emission signal each have relatively similar wavelengths. This can result in misinterpreting the emission signal, which misinterpretation results in an inaccurate determination of the analyte concentration. It would be advantageous to provide a sensing system in which the wavelengths of the excitation and emission signals are substantially different.

While substantial differentiation of the excitation and emission signals can be achieved using phosphorescent organic indicators, dye stability is often a problem. Indeed, U.S.-A-3,725,648, explicitly excludes phosphorescent molecules as potential sensor materials because of the stability problems. Some phosphorescent platinum group inorganic complexes and phosphorescent lanthanide complexes have shown suitable stability but are quite expensive and may generate significant amounts of highly reactive singlet oxygen upon irradiation in the presence of oxygen. It would be advantageous to provide a sensing system in which substantial differentiation of excitation and emission signals is achieved using stable dyes with reduced, or no, generation of reactive species, such as singlet oxygen.

Substantial differentiation of the excitation and emission signals can be achieved with an excited singlet state by using energy transfer from an analyte responsive first dye to an analyte insensitive second dye (EP-A-0/381,026). Furthermore, pyrene excimer emission has been successfully employed in solutions for the detection of organic compounds (Ueno et al, Anal. Chem., 1990, 62, 2461-66) and anesthetics (Merlo et al, IEEE Engineering in Medicine & Biology, 11th Annual International Conference Proceedings, 1989), with excimer fluorescence serving as a reporter of the aggregation of cyclodextins or of a change in the local viscosity, respectively. These sensing mechanisms work on the premise of an analyte suppressing or enhancing the efficiency for population of an emissive excited state. These systems are not disclosed as being usable in solid sensing elements. An additional problem with such systems is that they are incompatible with phase modulated detection methods, since the lifetime of the emissive state is substantially independent of analyte concentration.

It would be advantageous to provide a sensor system in which the lifetime, and preferably both the lifetime and intensity, of the differentiated emitted signal are sensitive to analyte concentration in a medium. Emissions, for example, fluorescent emissions, which are sensitive or dependent in terms of both lifetime and intensity to analyte concentration are said to be dynamically quenchable by the analyte.

Fiber-optic based sensors are very useful, for example, in medical applications. One problem which may exist with such systems is related to the inherent flexibility of optical fibers. These flexible fibers have a tendency to bend which, in turn, distorts the signals being transmitted by the fibers to the signal processor. Signal distortion caused by fiber bends or other sensor system problems result in inaccurate concentration determinations. It would be advantageous to provide a sensor and concentration determination method which provide accurate concentration data in spite of such distortions.

U.S.-A-4,548,907 discloses a fluorescence-based optical sensor which includes a fluorophor having an acid form and a base form. The fluorophor is chemically unstable in the presence of the medium. Specifically, the relative amounts of the acid form and base form vary depending on the pH of the medium. The fluorophor is excited at two different wavelengths, one for the acid form and one for the base form, and fluorescence signals at a single wavelength are

detected. By ratioing the fluorescence signals obtained at the two different excitation wavelengths, the pH of the medium can be determined. This sensor has the advantage of using a single fluorophor. However, the sensor of this patent is limited in that only those analytes which influence the ratio of acid form to base form of the fluorophor can be monitored. Again, the emission lifetimes are substantially independent of analyte concentration. Also, no other multiple state optical indicators are taught or suggested.

Lee, et al in "Luminescence Ratio Indicators for Oxygen", Anal. Chem., 59, p 279-283, 1987, report on work the goal of which was to develop a single reagent that would show two luminescence bands, a shorter wavelength "analytical" band subject to quenching by oxygen and a longer wavelength "reference" band independent of oxygen levels. Specifically, the work was to formulate a system showing both shorter wavelength oxygen-sensitive pyrene monomer emission and longer wavelength oxygen-insensitive pyrene dimer emission. This work did not succeed in finding a ratio-based indicator system to measure oxygen in aqueous systems. Further, as noted above, using a shorter wavelength oxygen sensitive emission can result in oxygen concentration determination inaccuracies because of possible overlapping between this short wavelength emission and the excitation signal.

CA-A-2,015,415 discloses an oxygen sensor including a single species of indicator selected from perylene derivatives dispersed or immobilized in a crosslinked polydimethylsiloxane matrix which gives a shorter wavelength oxygen sensitive emission and a longer wavelength oxygen insensitive emission and, thus, can be used as both the indicator and the reference element. Using shorter wavelength oxygen sensitive emission can result in inaccuracies because of overlap with the excitation signal, as described above. Also, there is no teaching or suggestion that the shorter and longer wavelength emissions are the result of different forms of the indicator. To the contrary, the document implies that a single indicator species provides an oxygen-sensitive emission region and a different oxygen-insensitive emission region.

EP-A-0363219 discloses an oxygen sensing apparatus using Europium or Erythrosin-B as phosphors which are excited with a monochromatic light that is sine wave modulated in the kHz regime. The emitted light of a different wavelength is also sine wave modulated, with the phase difference between the two sine waves being a measure of the quenching effect of oxygen and, thus, a measure of the partial pressure of oxygen. This publication does not disclose the use of any other indicators, for example, fluorescent indicators. Modulation in the kHz region cannot be extended to shorter lived fluorescent indicators because the phase offsets introduced by transmission of the excitation and emission signals, e.g., through an optical fiber, become significant. Also, there is no teaching or suggestion that the phosphors used produce different emitting forms. Further, as noted above, the use of phosphorescent indicators can result in other problems.

Sharma et al in "Unusually Efficient Quenching of the Fluorescence of An Energy Transfer-Based Optical Sensor for Oxygen", Analytica Chimica Acta, 212 (1988) 261-265, discloses a two fluorophore system consisting of pyrene as energy donor and perylene as energy acceptor dispersed in silicone rubber. A strong fluorescent signal, which is sensitive to oxygen quenching, is emitted at 474 nm, where pyrene does not fluoresce but where the relatively oxygen insensitive perylene does fluoresce. This emitted signal is believed to result in part from a mixed pyrene/perylene excimer. No covalent bonding of pyrene and perylene to the silicon rubber is disclosed so that there is not teaching or suggestion as to how such covalent bonding affects the system. Also, since perylene fluoresces at the same wavelength as does the mixed excimer, the signal emitted from the perylene may interfere with the signal from the mixed excimer. It would be advantageous to employ an emitted signal for analyte concentration determinations which is substantially removed or resolved from other emissions in the system.

ANALYTICA CHEMICA ACTA, vol. 212, pages 261-265, discloses an optical sensor based on a transfer of electromagnetic energy from a donor to an acceptor. Energy transfer is a collisionless process that occurs through space and requires substantial optical overlap between the characteristic emission of the first dye and the characteristic absorption of the second dye. The first dye absorbs light at a characteristic excitation wavelength and its excited state energy is transferred to the second dye. In this way, the second dye can emit at its own characteristic emission wavelength in response to excitation of the first dye. oxygen, in such systems, is believed to quench the excited state of the first indicator (e.g. pyrene) thereby reducing energy transfer efficiency and the resultant emission from the second indicator (e.g., perylene).

It is the object of the present invention to provide a sensor and a method for sensing the concentration of a component or analyte of interest in a medium, which sensor or method give accurate, reliable, and reproducible concentration determination.

According to the invention, this object is solved by the subject matter of claims 1, 14, 20, and 21, respectively. The subject matter of the subclaims refers to preferred embodiments.

As used herein, the term "monomeric indicator component" refers to a species which provides a signal, preferably an optically detectable signal, in response to being exposed to an excitation signal, preferably an excitation light signal. A "fluorescent monomeric indicator component" is a monomeric indicator component which provides a fluorescence signal in response to being exposed to an excitation signal.

As used herein, the term "excimer component" refers to an excited state species derived from a molecular or sub-molecular interaction of two or more, preferably two, monomeric indicator components, preferably fluorescent monomeric indicator components, which have the same indicator structure. Isomers and tautomers of the same monomeric indicator components are included.

As used herein, the term "mixed excimer component" refers to an excited state species other than an excimer component derived from a molecular or sub-molecular interaction of two or more, preferably two, monomeric indicator components one or more, preferably one, of which is a donor component and one or more, preferably one, of which is an acceptor component and which have sufficiently similar electronic energy levels, ionization potentials and electron affinities to satisfy the following relationship:

$$E_D^{ox} - E_A^{red} > h\upsilon\ (hex) + 0.25eV$$

where $E_D^{ox}$ is the ground state oxidation potential of the donor component, $E_A^{red}$ is the ground state reduction potential of the acceptor component, each measured relative to the same standard, $h\upsilon$(hex) is the maximum energy in eV (electron volts) of the excited state species measured in n-hexane, and the donor component and acceptor component are assigned so that the absolute value of $E_D^{ox} - E_A^{red}$ is minimized.

The present invention provides sensors and methods for sensing the concentration, for example, partial pressure, of a component or analyte of interest, such as oxygen, in a medium, for example, an aqueous-based medium, such as blood. The present systems function to give accurate, reliable and reproducible concentration determinations. In addition, such determinations can be provided in spite of signal transmission problems, such as, bent optical fibers, and other operational difficulties which may affect the quality of the signals being transmitted. Further, efficient utilization of the indicator component or components is achieved often resulting in sensors having reduced indicator component loadings. Moreover, these advantageous results are achieved using sensors, equipment and techniques which are relatively simple, easily operated and conveniently executed.

In one broad aspect, the present sensors comprise a sensing element, an excitation assembly and a detector assembly. The sensing element includes one or more, preferably one or two, monomeric indicator components, preferably located in, more preferably covalently bonded to, a matrix material, preferably a solid matrix material. Each of these monomeric indicator components is capable of providing a first emitted signal of a given wavelength in response to being exposed to a first excitation signal. Further, this sensing element is capable of providing a second emitted signal, preferably having a longer wavelength than the first emitted signal or signals, in response to being exposed to a second excitation signal. Preferably, the first excitation signal or signals and the second excitation signal are the same signal. The second emitted signal is provided by an excimer component or a mixed excimer component produced in the sensing element from the monomeric indicator component or monomeric indicator components and is more dependent on, i.e., varies in response to changes in, the concentration of the analyte in the medium to which the sensing element is exposed than the first emitted signal or signals. That is, for example, the excimer component or mixed excimer component derived second emitted signal is dynamically quenched or subject to being dynamically quenched by the analyte to a greater extent than the first emitted signal or signals. Excimer component and mixed excimer component emissions which are dynamically quenchable are preferred because, for example, they are useful in both intensity-based and phase modulated sensing systems.

The excitation assembly is positioned and adapted to provide one or more excitation signals to the sensing element. Such excitation signals preferably each have a wavelength which is shorter than the first emitted signal or signals. The first and second excitation signals may each have substantially the same wavelength.

The detector assembly is positioned and adapted to detect either the second emitted signal from the sensing element, or the first emitted signal or signals and the second emitted signal from the sensing element.

The processor assembly is preferably positioned and adapted to process or analyze either the second emitted signal in determining the concentration of the analyte in the medium, or the first emitted signal or signals and the second emitted signal in determining the concentration of the analyte in the medium.

Using the excimer or mixed excimer provided second emitted signal as at least part of the basis for the analyte concentration determination provides substantial benefits. This second emitted signal is different and distinct from the first emitted signal or signals provided by the monomeric indicator component or components. This second emitted signal has a relatively long wavelength and, thus, is further shifted away (red-shifted) from the excitation signal or signals than is the first emitted signal or signals. Thus, there is reduced risk of misinterpreting the second emitted signal (for example, because of interference from the excitation signal or signals) than of misinterpreting the relatively shorter wavelength first emitted signal or signals. Employing longer wavelength excimer or mixed excimer provided second emitted signals which are more analyte sensitive than the first emitted signal or signals provided by the monomeric indicator component or components results in increased indicator utilization efficiency. That is, the presence of such analyte sensitive longer wavelength second emitted signal means that the formation of the excimer component or mixed excimer component is advantageously kinetically favored so that relatively less monomeric indicator component

or components are needed to form the same amount of excimer component or mixed excimer component (relative to a system using shorter wavelength emissions which are more analyte sensitive). Thus, the amount of monomeric indicator component or components can be reduced and/or even the size of the sensor can be reduced using the longer wavelength analyte sensitive emitted signals of the present invention. The use of the excimer or mixed excimer provided second emitted signal results in accurate, reliable, reproducible and efficient analyte concentration determinations.

A particularly useful embodiment involves a sensing element which provides an intensity modulated, preferably a sine wave modulated, second emitted signal in response to being exposed to an intensity modulated, preferably a sine wave modulated, second excitation signal. This modulated second emitted signal is provided by an excimer component or mixed excimer component produced in the sensing element. The processor assembly is positioned and adapted to analyze the modulated second emitted signal and the modulated second excitation signal in determining the concentration of the analyte in the medium. The extent of the phase shift between the modulated second excitation signal and the modulated second emitted signal and/or the magnitude of relative demodulation is/are dependent on the concentration of the analyte in the medium. As used herein, the term "relative demodulation" refers to the demodulation factor for the modulated emitted signal with respect to the modulated excitation signal which results in the modulated emitted signal. Specifically, in this instance, relative demodulation is the demodulation factor for the modulated second emitted signal with respect to the modulated second excitation signal. The use of the excimer component or mixed excimer component provided modulated second emitted signal as a basis for analyte concentration determinations provides substantial benefits, for example, in terms of very good analyte concentration accuracy determination. The modulated signals referred to herein may be modulated in the MHz range, as opposed to being limited to the kHz range as disclosed in the European Patent Publication noted above. The use of the extent of the phase shift and/or the magnitude of relative demodulation, as described herein, at a variety of modulation frequencies can be used to determine analyte concentration, and is included within the scope of the present invention.

In one embodiment, the processor assembly is adapted to determine the ratio of the second emitted signal to the first emitted signal or one of the first emitted signals (or vice versa). Such a signal ratio is itself preferably dependent on the concentration of the analyte in the medium. The first emitted signal or signals may also be dependent on the concentration of the analyte in the medium.

In another embodiment, the first emitted signal or signals and the second emitted signal are intensity modulated, preferably sine wave modulated, and the processor assembly is adapted to determine at least one of the extent of the phase shift between and the ratio of demodulation factors of the modulated first emitted signal or one of the modulated first emitted signals and the modulated second emitted signal. The extent of this shift and/or this ratio is/are dependent on the concentration of the analyte in the medium.

Using such a signal ratio or both the modulated first emitted signal or signals and the modulated second emitted signal in determining the analyte concentration reduces, or even substantially eliminates, the detrimental effect on the accuracy of the concentration determination caused by, for example, distortion in the signals, for example, as the result of bent optical fibers. One particular advantage of excimer component or mixed excimer component forming systems over internally referenced systems containing a separate second indicator dye is that the analyte sensitive emission and the reference emission can be well resolved from one another using a single excitation wavelength. Furthermore, the intensity ratio, or the phase difference or the demodulation factor ratio methods can provide linear Stern-Volmer calibration slopes

The use of modulated signals, as described herein, is intensity independent. That is, the use of an analyte sensitive extent of shift in modulated signals is applicable regardless of the intensity of the signals used. The use of such modulated signals provides for very reliable and reproducible analyte concentration determinations.

For the specific embodiments described herein, the excitation assembly, detector assembly and processor assembly may be chosen from equipment which is conventional and well known in the art.

In yet another broad aspect, the invention involves methods for sensing the concentration, for example, partial pressure, of an analyte, for example, oxygen or other normally gaseous component, in a medium, such as blood or other aqueous, for example, liquid aqueous, medium. These methods comprise: exposing a sensing element, as described herein, to the medium; causing the sensing element to provide a second excimer component or mixed excimer component provided emitted signal, or first monomeric indicator component provided emitted signal or signals and a second excimer or mixed excimer component provided emitted signal, as described herein; and analyzing the second emitted signal or at least one of the first emitted signals and the second emitted signal in determining the concentration of analyte in the medium. The excitation signal or signals may also be analyzed in these methods.

A still further broad aspect of the present invention is the provision of compositions, for example sensing elements, useful for measuring the concentration of an analyte in a medium. In one embodiment, the compositions comprise a solid matrix material which is permeable to the analyte in the medium, and an effective amount of an indicator component in, for example, within and/or on, preferably within, the matrix material. The indicator component includes a first species capable of providing a first emitted signal of a first wavelength in response to being exposed to a first

excitation signal, and a second species capable of providing a second emitted signal of a second given wavelength in response to being exposed to a second excitation signal. The first species is covalently bonded to the second species, preferably by a linkage which facilitates the appropriate interaction between the first and second species. The indicator component is covalently bonded to the solid matrix material, if at all, through a single covalent linkage. The composition is capable of providing a third emitted signal in response to being exposed to a third excitation signal. This third emitted signal is provided by an excimer component or a mixed excimer component produced from the first species and the second species. The third emitted signal is dependent on the concentration of the analyte in the medium. This third emitted signal is preferably dynamically quenchable by the analyte in the medium.

At least one of the first emitted signal and the second emitted signal is preferably also dependent on the concentration of the analyte in the medium. Preferably, the third emitted signal is dependent on the concentration of the analyte in the medium to a greater extent than is the first emitted signal and the second emitted signal. The indicator component is preferably covalently bonded to the solid matrix material. In a particularly useful embodiment, the solid matrix material is a silicone-based polymer. The first species and second species are preferably selected from polynuclear aromatic species and mixtures thereof.

A particular example of such indicator components are those systems in which two fluorescent dye species, either similar or different dye species, are covalently tethered to each other in such a manner to facilitate the production of the desired excimer component or mixed excimer component. In one particularly useful embodiment, the number of atoms, for example, carbon atoms, in the chain covalently linking the first and second species together is in the range of about 1 to about 20, more preferably about 2 to about 7.

As noted above, it is important that the excimer component and mixed excimer component provided emitted signals be dependent on the concentration of the analyte in the medium, preferably to a greater extent than the other emitted signal or signals from any given sensor of the present invention. Such excimer component and mixed excimer component provided emitted signals are preferably dynamically quenchable by the analyte in the medium. The other emitted signal or signals from any given sensor in accordance with the present invention may also be dynamically quenchable by the analyte in the medium. In one embodiment, the signal or signals emitted from the sensing element preferably result from the sensing element fluorescing. In this embodiment, the Stern-Volmer quenching constant of the excimer component or mixed excimer component provided emitted signal is preferably higher than that of the other emitted signal or signals.

The sensors, methods and compositions of the present invention are useful in applications where the analyte is not a dynamic quencher of the emitted signal or signals, but instead, where the analyte concentration can be made to affect the concentration of a messenger species which is capable of dynamically quenching the emitted signal or signals. For example, it is well known in the art that enzymatic reactions involving glucose, adenosine triphosphate (ATP) or cholesterol dependent production or consumption of oxygen enable oxygen optrodes to serve as transducers for detection of these analytes.

Although the monomeric indicator component may be physically mixed or dispersed in a matrix material of the sensing element, it is preferred that the monomeric indicator component or components be covalently bonded to the matrix material. For example, the monomeric indicator component or components may be covalently bonded to, and therefore an integral part of, the polymeric material which is preferably included in the matrix material. Although the monomeric indicator component or components may be part of a polymer molecule which includes more than one indicator component moiety, such monomeric indicator component or components are still considered monomeric since each such component is capable of providing an emitted signal which is characteristic of the basic indicator compound (monomer) from which it is derived. In contrast, the emitted signal provided by the excimer component or mixed excimer component produced in the sensing element from two or more individual monomeric indicator component moieties, for example, on the same polymer molecule and/or on different polymer molecules, has a different characteristic than the emitted signals from the monomeric indicator component or components. The excimer component or mixed excimer component is preferably produced when an excitation signal is provided to the sensing element. The excimer component or mixed excimer component is believed to be inactive and/or to not be produced in the absence of the excitation signal.

In many instances, the covalently bonded monomeric indicator component is derived from an indicator compound or substance which itself is not suitable to be covalently bonded to the matrix material. In these instances, it may be necessary to derivatize or functionalize such indicator compound and produce a precursor of the monomeric indicator component. This is done by chemically modifying the indicator compound to include at least one group with a functional portion, preferably a functional multiple bond, which functional portion is capable of chemically reacting with the matrix material or precursor of the matrix material to covalently bond the monomeric indicator component thereto. Of course, if the basic compound from which the monomeric indicator component or components is derived is able to be covalently bonded to the matrix material or matrix material precursor, it is not necessary to further derivitize or functionalize such compound.

The functionalizing of such non-covalently bondable indicator compounds is illustrated by selecting a matrix material comprising a silicone-based polymer and an indicator compound which is a polynuclear aromatic compound. Such polynuclear aromatic compounds include the basic (underivatized) polynuclear aromatic compounds, as well as one or more derivatives thereof, that is one or more derivatives including non-functional groups which do not react with the matrix material or matrix material precursor. Such indicator compounds are not able to be covalently bonded to such silicone-based polymers or their precursors. However, these indicator compounds can be reacted to form attachment groups having a functional portion, such as appropriately configured alkenyl groups, substituted alkenyl groups and the like, which are capable of reacting with and bonding to silicone-based polymers and/or precursors thereof, such as polymethylhydrosiloxanes. In particular, if the silicone-based polymer is to be derived by addition curing, it is preferred to functionalize or derivatize the indicator substance or compound to attach one or more alkenyl groups and/or substituted alkenyl groups thereto. Such groups are capable of being hydrosilylated, for example, with a polymethylhydrosiloxane to covalently bond the monomeric indicator component to the silicone-based polymer precursor. The resulting precursor or compound can be reacted with vinyl-terminated polysiloxane, thereby forming an addition-cure silicone including the monomeric indicator component.

Any type of group may be attached to the indicator compound, provided that such group is with a functional portion, preferably a multiple bond, more preferably a carbon-carbon multiple bond and still more preferably a carbon-carbon double bond, which is capable of chemically reacting with a polymer or polymer precursor to form the covalently bonded monomeric indicator component. The group also should have substantially no undue detrimental effect on the analyte sensitivity of the excimer component or mixed excimer component provided emitted signal, on the other emitted signal or signals used in making analyte concentration determinations, or on the medium to which the monomeric indicator component is exposed. Preferably, the group is organic in nature. Particularly attractive benefits are obtained when the monomeric indicator component is derived from a monomeric indicator component precursor including at least one indicator compound or substance which has an aromatic ring to which is directly covalently bonded a group with a functional multiple bond which is isolated by a silicon-free chain (i.e., a chain of atoms linking the aromatic ring to the functional multiple bond which includes no silicon atoms), more preferably a chain linking only carbon atoms, from the aromatic ring. Such indicator component precursors are relatively easy to produce and use, and provide sensing elements with very useful properties. In another particularly useful embodiment, the group is a vinyl group covalently bonded directly to an aromatic ring of the indicator compound.

As noted above, particularly useful groups include alkenyl groups; substituted alkenyl groups and the like. Such groups and substituted groups preferably include 2 to about 20 carbon atoms, and may have a terminal double bond, i.e., a double bond associated with the terminal carbon atom. Examples of useful groups include vinyl, allyl, butenyl, hexenyl, heptenyl, octenyl, decenyl and the like groups. The presently useful substituted alkenyl groups include the groups described herein substituted with one or more substituent groups including elements such as oxygen, nitrogen, carbon, hydrogen, silicon, halogen, phosphorus and the like and mixtures and combinations thereof. Thus, the attached group can include at least one heteroatom.

Various chemical modification techniques, many of which are conventional and well known in the art, may be employed to functionalize or derivatize the indicator compound with the group or groups to produce the monomeric indicator component precursor. Care should be exercised to avoid destroying or even substantially diminishing the analyte sensitivity (e.g., to the gas component of interest) and intensity of the presently useful emitted signal or signals in the process of attaching one or more groups. However, it has been found that sufficient sensitivity is maintained if the characteristic structure of the indicator compounds remains substantially unaffected, i.e., intact, after the chemical modification.

In a particularly useful embodiment, the indicator compound is sensitive to the concentration of oxygen and is one or more polynuclear aromatic compounds and/or one or more derivatives thereof. The polynuclear aromatic compound is preferably any fluorescent or absorbent, more preferably fluorescent, optical indicator of the polynuclear aromatic class. The polynuclear aromatic compound from which the indicator component is derived is still more preferably selected from the group consisting of perylene, derivatives of perylene, decacyclene, derivatives of decacyclene, benzoperylene, for example, benzo[ghi]perylene, derivatives of benzoperylene, for example, derivatives of benzo[ghi]perylene, coronene, derivatives of coronene, pyrene, derivatives of pyrene, porphycine, derivatives of porphycine, porphyrin, derivatives of porphyrin, chlorin, derivatives of chlorin, pthalocyanine, derivatives of pthalocyanine and mixtures thereof. Since perylene and derivatives of perylene have a relatively reduced sensitivity to oxygen, other polynuclear aromatic compounds, such as those noted herein, are preferably employed when the analyte is oxygen. When an excimer component is to be utilized, the monomeric indicator component is preferably selected from one polynuclear aromatic compound, derivatives of the same one polynuclear aromatic compound and mixtures thereof. Excellent results are achieved if the one polynuclear aromatic compound is benzo[ghi]perylene.

If desired, the basic polynuclear aromatic compound may be derivatized with one or more other groups, e.g., non-functional substituent groups such as alkyl groups, provided such derivatization does not substantially interfere with excimer component or mixed excimer component provided emitted signal generation. Such derivatives are discussed in U.S.-A-4,849,172 which is hereby incorporated in its entirety by reference herein. One goal of the use of such

derivatives is to increase the solubility of the indicator substance in the matrix material. The "covalent bonding" feature described herein mitigates against this solubility constraint. Thus, the basic or underivatized polynuclear aromatic compounds, e.g., as described herein, may be advantageously used to produce the covalently bonded monomeric indicator component. When the covalently bonded monomeric indicator component is derived from a polynuclear aromatic compound (even an underivatized polynuclear aromatic compound) it is herein considered a derivative of a polynuclear aromatic compound because the polynuclear aromatic compound may include at least a portion of the attached group, noted above, and is derivatized by being covalently bonded to the matrix material. Thus, for example, the monomeric indicator component of a sensing element derived by covalently bonding vinyl benzo[ghi]perylene in an addition cure silicone polymer is said to be a derivative of benzo[ghi]perylene.

Preferably, the excimer component and mixed excimer component producing monomeric indicator components of the present sensing elements are positioned or oriented so as to facilitate excimer component or mixed excimer component formation. Thus, these monomeric indicator components can be said to be in enforced association. By "enforced association" it is meant that the monomeric indicator components are positioned or oriented in physical and/or molecular enforcement to promote or facilitate the formation and/or maintenance of a channel or path for excimer component or mixed excimer component formation which (a) is kinetically dominant relative to free diffusion, and/or (b) is kinetically competitive with the decay of the excimer component or the mixed excimer component in the absence of the analyte. Enforced association facilitates the development of sensors based on a wide variety of monomeric indicator components which may not otherwise form appreciable excimer component or mixed excimer component at similar concentrations as free monomeric indicator components. Enforced association provides relatively long lived excimer components and mixed excimer components with acceptable Stern Volmer slopes in cases where the monomeric indicator component or components themselves are too short lived to be useful. Enforced association may minimize kinetic heterogeneity for excimer component and mixed excimer component dissociation, thereby affording more reproducible Stern Volmer slopes based on the excimer component or mixed excimer component to monomeric indicator component ratio or the phase shift difference or the demodulation factor ratio or on the excimer component emission or mixed excimer component emission alone.

Enforced association can be achieved in various ways, for example, by:

1. Intramolecular excimer component or mixed excimer component forming molecules dispersed in a matrix material.
2. Intramolecular excimer component or mixed excimer component forming molecules covalently attached to a matrix material.
3. Monomeric indicator component aggregates dispersed in or covalently attached to a matrix material or adsorbed, preferably at high loadings, to the surface of a matrix material.
4. Intramolecular excimer component or mixed excimer component forming molecules covalently attached, adsorbed or otherwise attached to the surface of a matrix material.

A particularly useful "monomeric" indicator component is one which includes a first indicator species and a second indicator species which are covalently bonded together. Each of these species may be considered a monomeric indicator component itself since each is capable of emitting a signal in response to being excited, and each is capable of combining with another monomeric indicator component to produce an excimer component or a mixed excimer component. By covalently bonding or tethering the two species together, the two species are positioned to have increased accessibility to each other for excimer or mixed excimer formation. The covalent link between the two species is preferably such that the formation of an excimer component or a mixed excimer component from the two covalently linked species is facilitated. Such covalent link, particularly where each of the species is a polynuclear aromatic species, such as described above, preferably includes about 1 to about 20, more preferably about 2 to about 7, atoms, for example, carbon atoms, in the chain between the two species.

The indicator component including the first and second indicator species, as described above, is covalently bonded, if at all, to a solid matrix material through a single covalent linkage. Thus, such indicator components do not include monomeric indicator components which are each separately covalently bonded to a polymeric material, for example, the present matrix material.

The covalent link between the two species can include a functional portion to enable the indicator component to be covalently linked through a single covalent link to the solid matrix material.

The use of bichromophoric components such as the covalently bonded or tethered indicator components described above, provides for reproducible and reliable analyte concentration determinations by generating reproducible working curves which are independent of bichromophoric component concentration over a broad concentration range.

The amount of monomeric indicator component or components (or indicator component or components) employed in the present systems may vary over a broad range and depends, for example, on the particular component or components being employed, on the matrix material employed, on the sensing application involved and the like. Such

amount or amounts should be effective to produce the desired excimer component or mixed excimer component and to yield the desired signal or signals. For example, the amount of monomeric indicator component or components (or indicator component or components) may be in the range of about 0.0001% or about 0.001% to about 10% or about 20% or more, by weight calculated on the total weight of the sensing element. In many instances, concentrations of less than about 5% or even about 1% or less, by weight calculated on the total weight of the sensing element, provide excellent results. Care should be exercised, for example, if it is desired (as it is preferred) to achieve enforced association, to avoid conditions which result in extended aggregates or extended aggregation of such component or components which can cause additional quenching of the excimer component or mixed excimer component emission (independent of the analyte concentration) and/or a reduction in analyte sensitivity of the excimer component or mixed excimer component emission.

Any suitable matrix material, preferably a polymeric matrix material, may be employed provided that it functions as described herein. Particularly useful polymeric matrix materials include those based on addition cure silicone polymers. The matrix material, or the precursor thereof, should preferably be such as to chemically react with the precursor or precursors of the monomeric indicator component or components and produce a sensing element with covalently bonded monomeric indicator component or components.

Although various polymers can be employed as the matrix material, it is preferred that the matrix material be permeable, more preferably highly permeable, to the analyte, for example, a normally gaseous component, of interest so that the sensitivity of the sensing element to the analyte of interest is optimized. If a silicone-based polymer is employed in the matrix material, it may include polymers derived from vinyl terminated polysiloxanes and polyalkyl (aryl)hydrosiloxanes. Such polyalkyl(aryl) hydro siloxanes include, but are not limited to, those having the formula

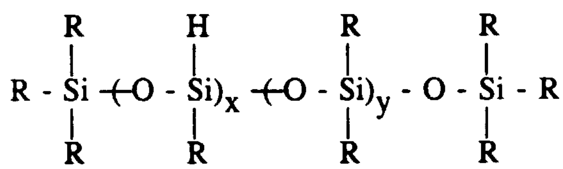

where each of x and y is independently an integer in the range of 1 to about 500 and R is independently selected from the group consisting of H, alkyl, and substituted alkyl, preferably $CH_3$, $CH_2$ $CH_2$ $CF_3$, $CH_2(CH_2)_nCH_3$, and phenyl, where n is an integer in the range of 1 to about 22. Of this group, polymers in which a major portion of the R groups are methyl are preferred because of the high gas permeability of such polymers. A sufficient number of hydride groups should be present to provide a satisfactory cross-linked or cured polymer, and preferably to react with the monomeric indicator component precursor to covalently bond the monomeric indicator component to the matrix material. It is of course realized that other members of the homologous series which include the above-noted polymers may also be used. The final silicone-based matrix material is cross-linked. Suitable vinyl terminated polysiloxanes include two or more functional vinyl groups which react with the hydride or hydro groups of the polyalkyl(aryl)hydrosiloxanes, for example, polymethylhydrosiloxanes, to form the cross-linked matrix material. Such cross-linking advantageously occurs in the presence of a catalyst, such as a platinum-containing catalyst. The properties of the cross-linked silicone can be varied by changing the degree of cross-linking, for example, by adjusting the concentration of the Si-H groups or component on the polyalkyl(aryl)hydrosiloxanes, for example, polymethylhydrosiloxanes and/or the molecular weight of the vinyl-terminated polysiloxanes.

The precursors of the monomeric indicator components useful in the present invention can be obtained using synthesizing procedures, such as formation of aldehydes, Wittig reactions to give vinyl derivatives, and the like. The monomeric indicator component precursor or precursors thus obtained can be dispersed in a silicone-based polymer, such as polymethylhydrosiloxane, in a volatile solvent, such as benzene, hexane and the like, and be allowed to react to covalently bond the monomeric indicator component or components to the silicone-based polymer. The silicone-based polymer, having the chemically attached indicator component or components, is then reacted, for example, using conventional addition curing, to form the sensing element.

An alternative, and less desirable, method for producing the present sensing element involves combining the precursor or precursors of the monomeric indicator component or components with the vinyl-terminated polysiloxane, preferably in an inert solvent to promote dissolution of the above-noted precursor or precursors, and any catalyst e.g., platinum group metal, which may be employed. This combination is then mixed with polymethylhydrosiloxane at conditions effective to covalently bond the monomeric indicator component or components to the matrix material and form the present sensing element.

These and other aspects and advantages of the present invention are set forth in the following detailed description and claims, particularly when considered in conjunction with the accompanying drawings.

Fig. 1 is a schematic illustration of one embodiment of the sensor apparatus according to the present invention.

Fig. 2 is a schematic illustration of an alternate embodiment of the sensor apparatus according to the present invention.

Fig. 1 shows a sensor 10 according to the present invention. Sensor 10 is adapted to determine the concentration or partial pressure of oxygen in blood. An optical fiber 12 is connected to an appropriate light transmitting apparatus 14, which is capable of transmitting light at 395 nanometers. The light transmitting apparatus 14 generates the excitation light at this wavelength. The optical fiber 12 is also connected to a light receiving apparatus 16, which, in turn, is connected to a conventional electronic processor 17.

Located on the optical surface 18 of the optical fiber 12 is a matrix 20 which is an oxygen permeable material, such as a cross-linked addition cured siloxane polymer. Covalently bonded in matrix 20 is about 1.0% by weight of a mixture of vinyl derivatives of benzo[ghi]perylene.

This siloxane polymer is obtained by reacting a polymethylhydrosiloxane, such as those described above in which x is equal to about 10 and y is equal to about 19, with a mixture of vinyl benzo[ghi]perylene derivatives to covalently bond the monomeric benzo[ghi]perylene moieties to the polymethylhydrosiloxane. This modified polymethylhydrosiloxane is than reacted with a vinyl terminated polysiloxane in the presence of a platinum catalyst to form a cross-linked siloxane polymer including covalently bonded monomeric benzo[ghi]perylene moieties. The vinyl terminated polysiloxane has the following formula

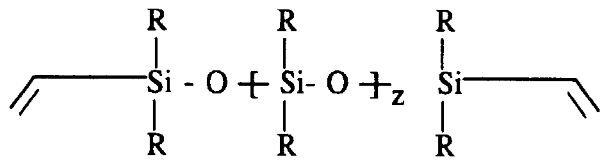

where z is about 376 and each R is methyl. The ratio of SiH to vinyl in the cross-linking reaction is controlled to provide a suitable cross-linked elastomer product.

The highly oxygen permeable matrix 20 adheres to the optical surface 18 and slightly down along the sides 22 of the end of fiber 12. An opaque overcoating 24, comprising iron oxide pigment dispersed in an addition cured polysiloxane, can then be applied over the totality of the matrix 20 and down further along the side 22 of the fiber 12.

In use, sensor 10 functions as follows. The tip of optical fiber 12 including matrix 20 and overcoating 24 is exposed or immersed in blood, the oxygen concentration of which is to be determined. Light transmitting apparatus 14 transmits light at 395 nanometers to the optical fiber 12. The excitation light at 395 nanometers causes the matrix 20 to fluoresce at two separate wavelengths, 421 nanometers and 460 nanometers. The emission at the first or shorter wavelength is the result of the excitation of monomeric benzo[ghi]perylene moieties in matrix 20. The emission at the second or longer wavelength is the result of an excimer which is formed by the interaction of one or more excited monomeric benzo[ghi] perylene moieties in matrix 20 and one or more unexcited (or ground state) benzo[ghi]perylene moieties in matrix 20. Both the emissions at 421 nanometers and 460 nanometers are dependent on the concentration of oxygen in the blood with the longer wavelength emission being more so dependent than the emission at 421 nanometers.

The fluorescent emitted signals are transmitted from matrix 20 through optical fiber 12 to light receiving apparatus 16. Processor 17 uses information received by light receiving apparatus 16 on the longer emitted signal to determine a value of the oxygen concentration in the blood. Receipt and analysis of this fluorescent light by light receiving apparatus 16 and processor 17 is carried out in a manner similar to that described in the above-referenced Lubbers, et al patent and in Heitzmann U.S. Patent 4,557,900 each of which is incorporated in its entirety herein by reference.

Processor 17 uses information received by light receiving apparatus 16 of the fluorescent signal emitted at 421 nanometers to develop a ratio of the emitted fluorescent signal at 460 nanometers to the fluorescent signal at 421 nanometers. Using this ratio together with the above-noted oxygen concentration, processor 17 can determine a corrected concentration of oxygen in the blood to be analyzed. This corrected oxygen concentration is found to be accurate even if the optical fiber 12 is bent at one or more points along its length and/or if other light transmission difficulties are encountered.

The above-noted procedure may occur periodically or even substantially continuously to give substantially continuous oxygen concentration results. Of course, the transmission of the emission at 421 nanometers can take place before transmission of the emission at 460 nanometers. Also, by proper selection of the optical indicators, e.g., fluorescent dyes, the concentration of other components of interest can be determined. In addition, media other than blood can be analyzed.

The optical fiber 12 may be in the form of a probe or a catheter insertable into a blood vessel of a patient to provide

continuous on-line in vivo monitoring of oxygen concentration in the blood. Alternately, the present sensor can be embodied in a flow-through housing as shown, for example, in the above-referenced Heitzmann patent, to provide extra corporeal monitoring of oxygen concentration in the blood.

In an additional embodiment, the matrix 20 is a highly oxygen permeable material, such as a cross-linked, siloxane-based polymer, and includes about 1.0% by weight of an indicator component derived from vinyl benzo[ghi]perylene covalently bonded to the polymer and about 1.0% by weight of vinyl perylene covalently bonded to the polymer. The opaque overcoating 24, comprises a mixture of carbon black and cellulosic material.

In use, this alternate embodiment of sensor 10 functions as follows. The tip of optical fiber 12 including matrix 20 and overcoating 24 is exposed or immersed in blood, the oxygen concentration of which is to be determined. Light transmitting apparatus 14 transmits light at 395 nanometers to the optical fiber 12. The excitation light at 395 nanometers causes the matrix 20 to fluoresce, which is believed to result in part from an excimer of benzo[ghi]perylene functionalities or moieties. The lifetime of this fluorescent signal, at a wavelength of about 450 nanometers, is longer than about 50 nanoseconds. A fluorescent signal is transmitted from matrix 20 through optical fiber 12 to light receiving apparatus 16. This fluorescent signal, derived from excitation light at 395 nanometers, depends on the concentration of oxygen in the blood being analyzed. Processor 17 uses information received by light receiving apparatus 16 on this fluorescent signal to determine a value of the oxygen concentration in the blood.

In a further additional embodiment, the matrix 20 is a highly oxygen permeable material, such as a cross-linked, siloxane-based polymer, and includes about 1.0% by weight of an indicator component derived from allyl benzo[ghi]perylene covalently bonded to the polymer and about 1.0% by weight of allyl perylene covalently bonded to the polymer. The opaque overcoating 24 comprises a mixture of carbon black and cellulosic material.

In use, this further alternative embodiment of sensor 10 functions as follows. The tip of optical fiber 12 including matrix 20 and overcoating 24 is exposed or immersed in blood, the oxygen concentration of which is to be determined. Light transmitting apparatus 14 transmits light at 395 nanometers to the optical fiber 12. The excitation light at 395 nanometers causes the matrix 20 to fluoresce, which emission is believed to result in part from an excimer of benzo[ghi]perylene functionalities or moieties. The lifetime of this fluorescent signal, at a wavelength of about 450 nanometers, is longer than about 50 nanoseconds. A fluorescent signal is transmitted from matrix 20 through optical fiber 12 to light receiving apparatus 16. This fluorescent signal, derived from excitation light at 395 nanometers, depends on the concentration of oxygen in the blood being analyzed. Processor 17 uses information received by light receiving apparatus 16 on this fluorescent signal to determine a value of the oxygen concentration in the blood.

An alternate embodiment, which is described with reference to Fig. 2, involves a sensor apparatus making use of intensity modulated (sine wave) signals in the MHz range.

In this embodiment, sensor 110 is adapted to determine the concentration or partial pressure of oxygen in blood. An optical fiber 112 is connected to an appropriate light transmitting apparatus 114, which is capable of transmitting intensity modulated (sine wave) light in the MHz range. The light transmitting apparatus 114 generates the modulated excitation light at this frequency. The optical fiber 112 is also connected to a light receiving apparatus 116, which, in turn, is connected to a conventional electronic processor 117.

The light transmitting apparatus 114 includes a frequency generator (one or more frequencies simultaneously) linked to an electrically controlled light emitting structure, such as a light emitting diode, a frequency doubled light emitting diode, or a combination of elements such as a continuous wave laser or incandescent light source coupled to an acoustooptic modulator or electrooptic modulator, and the like.

The light receiving apparatus 116 includes a highly sensitive light detector having a rapid response time. Suitable detectors include photomultiplier tubes such as those sold under the trademark R928 by Hamamatsu Photonics K.K., Hamamatsu, Japan, as well as avalanche photodiodes and microchannel plates, also available from the same supplier. Using techniques well known in the art, heterodyne detection can be implemented by modulating the detector sensitivity at a frequency, equal to the fundamental modulation frequency, $F_f$ in the MHz regime, plus or minus a heterodyne modulation frequency $F_h$ in the Hz or kHz region.

The processor 117 may include, for example, an analog to digital converter coupled by a direct memory access device to a computer, or an analog phase comparator circuit known to those skilled in the art, and the like. The SLM 48000MHF Fourier Transform Spectrofluorometer manufactured by SLM-Aminco in conjunction with an argon ion laser provides frequency modulated light generation, light receiving apparatus and processor capability to perform the methods outlined herein; to measure phase shifts, demodulation factors, or both at either a single modulation frequency or simultaneously at several modulation frequencies. Commercial software is available to apply a well-known digital fast Fourier transform to the data and to interpret phase and demodulation data at multiple modulation frequencies in terms of a distribution of emission lifetimes and relative contributions. This enables determination of the contribution of the excimer component, mixed excimer component and monomeric indicator component provided emitted signals to the total phase shift and/or demodulation factors at each wavelength, even when several overlapping emission signals are present.

Located on the optical surface 118 of the optical fiber 112 is a matrix 120 which is an oxygen permeable material,

such as a cross-linked addition cured siloxane polymer which is similar to the polymer described previously. Dispersed in the polymer is 0.004% by weight of 1,3-bis-(1-pyrene)propane commercially available through Molecular Probes Inc.

The highly oxygen permeable matrix 120 adheres to the optical surface 118 and slightly down along the sides 122 of the end of fiber 112. An opaque overcoating 124, comprising iron oxide pigment dispersed in an addition cured polysiloxane, can then be applied over the totality of the matrix 120 and down further along the side 122 of the fiber 112.

In use, sensor 110 functions as follows. The tip of optical fiber 112 including matrix 120 and overcoating 124 is exposed or immersed in blood, the oxygen concentration of which is to be determined. Light transmitting apparatus 114 transmits light at 10MHz and 325 nm to the optical fiber 112. This excitation light causes the matrix 120 to fluoresce at two separate wavelengths, 375 nanometers and 500 nanometers. Both fluorescent emissions are sine wave modulated. The emission at the first or shorter wavelength is believed to be the result of the emission from monomeric pyrene moieties in matrix 120. The emission at the second or longer wavelength is the result of an excimer which is formed by the interaction of one or more excited monomeric pyrene moieties in matrix 120 and one or more unexcited (or ground state) pyrene moieties in matrix 120.

The fluorescent emitted signals are transmitted from matrix 120 through optical fiber 112 to light receiving apparatus 116. Processor 117 uses information received by light receiving apparatus 116 on the emitted signals to determine the extent of the phase shift between and/or the ratio of demodulation factors of these two emitted signals. The extent of this phase shift and/or this ratio of demodulation factors are dependent on the concentration of oxygen in the blood. Thus, by determining the extent of this phase shift and/or this ratio of demodulation factors, values of the oxygen concentration in the blood can be obtained.

Alternately, or as a check on the oxygen concentrations obtained by analyzing the two emission signals, processor 117 can use information received from the light transmitting apparatus 114 on the excitation light and information received by light receiving apparatus 116 on the excimer-derived emitted signal to determine the extent of the phase shift and/or the magnitude of relative demodulation between this emitted signal and the excitation signal. The extent of this phase shift and/or this magnitude of relative demodulation are dependent on the concentration of oxygen in the blood. Thus, by determining the extent of this phase shift and/or this magnitude of relative demodulation, values for the oxygen concentration in the blood can be obtained.

Transmission, receipt and analysis of these modulated signals by light transmitting apparatus 114, light receiving apparatus 116 and processor 117 may be carried out using equipment and in a manner similar to that described in Gratton U.S. Patent 4,840,485 which is incorporated in its entirety herein by reference.

The above-noted procedure may occur periodically or even substantially continuously to give substantially continuous oxygen concentration results. Of course, by proper selection of the optical indicators, e.g., fluorescent dyes, the concentration of other components of interest can be determined. In addition, media other than blood can be analyzed.

The optical fiber 112 may be in the form of a probe or a catheter insertable into a blood vessel of a patient to provide continuous on-line in vivo monitoring of oxygen concentration in the blood. Alternately, the present sensor can be embodied in a flow-through housing as shown, for example, in the above-referenced Heitzmann patent, to provide extra corporeal monitoring of oxygen concentration in the blood.

The following non-limiting Examples illustrate certain aspects of the invention.

Fluorescence data were obtained on a SPEX Fluorolog Fluorometer, equipped with a 450W Xe lamp, single excitation monochromator blazed at 250 nm, double emission monochromator blazed at 500nm. Typical slit widths were 0.5mm or less corresponding to bandwidths on the order of 1 to 2 nm. Emission spectra were corrected and zeroed, excitation spectra were obtained in the ratio mode and were corrected. For variable (oxygen concentration) studies, films were placed in a flow-through chamber equipped with glass or quartz windows. The chamber volume was small to allow for fast gas exchange. The spectra were taken in a front-face mode with excitation normal to the film and emission collected at 22° to the film normal.

Phase modulation experiments were performed using a modified version of the commercially available SLM 48000 Frequency-Domain Fluorometer with single frequency acquisition or with Multi-Harmonic Fourier (MHF)Parallel Acquisition. The SLM 48000 sample chamber was modified by replacing the cuvette holder mount with an x, y, z translator. The proximal terminus of the fiber optic was positioned with the translator at the focal point of the excitation beam (which is focussed with a f/2 lens) such that it was perpendicular to the line of propagation of the excitation. Typically, 200, 600 or 1000 microns core diameter quartz multi-mode fiber optic cables (General Fiber Optics) were used to carry excitation to the sample chamber, and a second fiber of the same core diameter was used to carry emission to the detector. The fibers were cladded with black, electrical cladding to minimize false light effects. The fiber termini were polished and adapted with a Amphenol-type connector on the spectrometer end and a stainless steel capillary tube on the sample end.

EXAMPLE 1

Into a clean dry 500 ml round-bottom flask fitted with a drying tube, a rubber septum and a magnetic stirrer was

added 3.2 g of benzo[ghi]perylene and 400 ml of dichloromethane under a blanket of dry nitrogen. When the crystals had dissolved, the solution was cooled to 10° C. Using a 10 ml syringe, 7.5 g of tin (IV)tetrachloride was added. The mixture was made homogenous by stirring, then 4.5 g of dichloromethyl methyl ether was added over 2 minutes. The mixture became blue purple and viscous. The viscosity dropped after about 30 minutes of stirring. The reaction was complete after about 150 minutes. The reaction mixture was quenched with 600 ml of 2N HCl and the product extracted with 600 ml of dichloromethane. The organic layer was washed with 600 ml of water and dried over anhydrous sodium sulfate. The organic phase was passed through a 3.5" x 2" silica gel plug, and the product, benzo[ghi]perylene aldehyde in good yield and purity, was separated from the solvent with a rotary evaporator.

EXAMPLE 2

2.7 g of methyl iodide and 4.5 g of 4- (diisopropylaminomethyl)triphenyl phosphine in 10 ml of toluene were allowed to react in a 50 ml round-bottom flask equipped with a magnetic stirrer with stirring overnight at room temperature under dry nitrogen. The reaction mixture formed two phases. The toluene was removed by rotary evaporation. To the remaining mixture was added 12 mmols of lithium diisopropyl amide in anhydrous tetrahydrofuran at 0°C and stirred for 30 minutes at this temperature. This mixture was added to 3.1 g of benzo[ghi]perylene aldehyde dissolved in 300 ml of anhydrous tetrahydrofuran under an inert atmosphere (dry nitrogen or argon) at O°C. The reaction was over in an hour, at which time the solvent was removed by rotary evaporation. The residue was dissolved in 500 ml of toluene and washed with 2, 100 ml portions of 0.50 M HCl followed by a water wash and an aqueous 2.5% sodium bicarbonate wash. The organic phase was dried over sodium sulfate. The dry organic phase was stirred with 2 g of activated charcoal, then chromatographed on a 3" x 1" silica gel column that had been deactivated with ethanol. The product was eluted from the column with toluene. The product, a mixture of vinyl benzo[ghi]perylene isomers, was recovered by rotary evaporation of the solvent and stored in an amber bottle under nitrogen.

EXAMPLE 3

In a nitrogen purged 50 ml round-bottom flask equipped with a magnetic stirrer was placed the vinylbenzo[ghi]perylene product from Example 2 (131 mg), polymethylhydrosiloxane (1.0 g; sold by Petrarch Systems under the trademark PS123), and benzene (30 ml). Platinum catalyst (150 microliters diluted to 10% in hexane; sold by Petrarch Systems under the tradename PC075) was added at room temperature, and the reaction was brought to a gentle reflux. The reaction was determined to be complete by thin layer chromography, using a hexane/ether (90/10) mobile phase, when the yellow fluorescent spot (vinylbenzo[ghi]perylene; $R_f = 0.5$) was no longer present. The benzene was removed by rotary evaporation and replaced by hexane (30 ml). Subsequent to rotoevaporation of the hexane, the yellow oil was redissolved in hexane (30 ml) and decolorizing carbon (400 mg; sold by J.T. Baker Chemical Company under the trademark Darco G-60) was added. The mixture was gently stirred for 20 minutes then filtered using celite as an aid. Following rotoevaporation of the solvent, the product was adjusted to the appropriate concentration using hexane. This solution was then combined with a vinyl terminated siloxane and platinum catalyst to form an immobilized, cross-linked fluorescent silicone rubber.

The resulting cross-linked fluorescent silicone rubber includes an effective amount of chemically bound, non-leachable monomeric benzo[ghi]perylene moiety, and is effective as a sensing element in an in vivo blood oxygen sensor in accordance with the present invention, for example, as matrix 20 in sensor 10, as described above.

EXAMPLE 4

A series of addition cure silicone elastomers containing covalently bonded benzo[ghi]perylene moieties, derived from vinyl benzo[ghi] perylene were produced. The basic elastomer had substantially the same composition as matrix 20 except that the vinyl terminated polysiloxane used was such that z equaled about 78. Two other similar elastomers were produced except that one elastomer contained only 10% of the benzo[ghi] perylene moieties as did the basic elastomer, and another elastomer contained only 1% of the benzo[ghi] perylene moieties as did the basic elastomer.

Each of these elastomers was tested for fluorescent emission intensity upon being exposed to nitrogen gas containing varying oxygen concentrations and being excited with light at 395 nm. Emissions were monitored at 420 nm (believed to result from the monomeric benzo[ghi]perylene moieties) and at 450 nm or 480 nm (believed to result from excimer produced by the benzo[ghi]perylene moieties). Ratios of the intensities of various emissions were determined.

Results of these tests were as follows:

| ***Elastomer*** | ***$O_2$ Concentration (partial pressure) mm*** | ***Ratio(1) 480/420*** | ***Ratio(1) 450/420*** |
|---|---|---|---|
| BASIC | 0<br>160 | 1<br>1.27 | 1<br>1.18 |
| ELASTOMER CONTAINING 10% BENZO[GHI]PERYLENE MOIETIES OF BASIC ELASTOMER | 0<br>160 | 1<br>1 | 1<br>1.11 |
| ELASTOMER CONTAINING 1% BENZO[GHI]PERYLENE MOIETIES OF BASIC ELASTOMER | 0<br>160 | 1<br>1 | 1<br>1 |

(1) Ratio equals (E/M)o/(E/M) where (E/M)o is the ratio of the intensities of the excimer and monomer measured under nitrogen and (E/M) is the ratio of the intensities of the excimer and monomer measured at the oxygen concentration listed.

These results indicate that the excimer/monomer emission intensity ratio is dependent on the oxygen concentration when the concentration of monomeric indicator component is relatively high. Thus, at relatively high monomeric indicator component concentrations this emission intensity ratio can be used in determining oxygen concentration.

EXAMPLE 5

This is an example of the preparation of a film containing an intramolecular excimer component-forming moiety. To 0.50 g of a vinyl terminated siloxane (sold by Petrarch Systems under the trademark PS441) were added 0.05 g of a polymethylhydrosiloxane (sold by Petrarch Systems under the trademark PS123), 0.50 ml of a 9X $10^{-5}$ M $CH_2Cl_2$ solution of 1,3-bis-(1-pyrene)propane, and 10 microliters of Pt catalyst solution.

The Pt catalyst solution used in these preparations was a solution of Karsted's catalyst in hexane. The solvents used were spectral grade, dried over molecular sieves. 1,3-bis-(1-pyrene) propane was purchased from Molecular Probes, Inc., Eugene, OR.

The mixture was agitated and poured into a 57 mm diameter aluminum weighing pan and allowed to dry and cure in air, then further dried under vacuum overnight to remove residual solvent. The films thus obtained are optically clear.

The response of this film to oxygen was determined in the intensity mode. Upon being excited at 345 nm, excimer emission was monitored at 500nm and monomer emission at 375nm. Both excimer emission and monomer emission were dynamically quenchable by oxygen. When plotted in Stern-Volmer form there was obvious curvature of the excimer oxygen dependence. However, the excimer/monomer ratio in a Stern-Volmer treatment produced a linear response with a slope of $0.0125mm^{-1}$.

EXAMPLE 6

The response of the film produced in Example 5 was determined in phase. The film was placed in a thermostated optical isolation chamber equipped with a port for collinear excitation and emission optical fibers and ports for rapid gas exchange. The fiber optic port was positioned to allow the distal fiber termini to provide for excitation and emission collection at an angle of 45 degrees from the film surface to minimize scatter. The film was excited at 325nm using monochromatic excitation provided from an argon ion laser (Coherent Model 90-6). Emission wavelengths of 500 nm for excimer emission and 375 nm for monomer emission were serially selected using a standard monochromator. The sensor film was exposed to oxygen in nitrogen mixtures with the following composition (volume% oxygen = 0.0, 5.09, 10.35, 15.37, and air).

Uncorrected phase shifts were obtained from MHF or from single frequency data. These phase shifts were referenced to the excitation source phase and included phase offsets associated with the electronics and optics. For a modulation frequency of 10 MHz, uncorrected phase data, the difference between the excimer and monomer phase measurements, and the resultant Stern-Vomer slope for oxygen quenching of the excimer fluorescence deconvolved from the kinetics for indirect excimer population were as follows:

| | | | | | |
|---|---|---|---|---|---|
| mm $O_2$ | 0.00 | 38.7 | 78.7 | 117 | 159 |
| excimer phase | 244.41 | 228.54 | 221.34 | 215.22 | 219.04 |
| monomer phase | 169.73 | 159.27 | 157.09 | 154.54 | 164.82 |
| phase difference | 74.68 | 69.27 | 64.25 | 60.68 | 54.22 |
| $T_o/T=\tan\Delta\theta_o/\tan\Delta\theta$ | 1.00 | 1.38 | 1.76 | 2.05 | 2.63 |

A linear Stern-Volmer slope of 0.0099 was obtained.

Importantly, an equivalent linear Stern-Volmer slope can be obtained from the demodulation factors for the excimer ($m_e$) and for the monomer ($m_m$) in accordance with the following relationship: $T_o/T=\{[(m_m/m_e)_O{}^2-1]/[(m_m/m_e)^2-1]\}^{1/2}$ , where $T_o$ and T are lifetimes for the excimer emission in the absence and the presence of the analyte, respectively, deconvolved from the kinetics for indirect generation of the excimer from the monomer. Further, it should be noted that the equivalent linear Stern-Volmer slope can be obtained using the intensity ratio method.

EXAMPLE 7

This is an example of the preparation of a film containing a polymer-attached or covalently bonded intramolecular excimer component-forming moiety. To 0.05 g of polymethylhydrosiloxane (as described in Example 5) was added 0.50 ml of a $9x10^{-5}$ M $CH_2Cl_2$ solution of 1,5-bis-(1-pyrene)-3-pentanone, and 10 microliters of the Pt catalyst solution (as described in Example 5). This mixture was allowed to react for five minutes, then was added to 0.50 g of vinyl terminated siloxane (as described in Example 5). An additional 10 microliters of the Pt catalyst solution was added. The mixture was agitated and poured into a 57 mm diameter aluminum weighing pan and allowed to dry and curing in air, then further dried under vacuum overnight to remove residual solvent. The film thus obtained was optically clear.

This film when excited with a monochromic light, for example, at 345 nm, in the presence of varying concentrations of oxygen, provides both a monomer derived emission and a longer wavelength excimer component derived emission. Both emissions are dynamically quenchable by oxygen.

EXAMPLE 8

This Example illustrates the preparation of bichromorphic excimer-forming compounds which can be covalently attached to a polymeric, in particular a polysiloxane, matrix material, by hydrosilation of the ketone moiety.

1,5-bis-(1-pyrene)-2,4-pentadien-3-one

To a solution of 16 g NaOH in 50 ml $H_2O$ was added 1.0 ml tetrabutyl ammonium hydroxide titrant solution. A solution of 11.6 g 1-pyrene carboxaldehyde, 1.6 g acetone, and 50 ml $CH_2Cl_2$ was added to the basic solution; a dark orange precipitate formed immediately. The mixture was allowed to stir from 1 to 12 hours. The mixture was diluted with 100 ml $H_2O$ and 100 ml $CH_2Cl_2$ and filtered through a glass frit. The solid was washed with $CH_2Cl_2$, ethanol, methanol, and air dried. The solid was broken up, triturated with 100 ml $CH_2Cl_2$, filtered and air dried to yield 7.20 g 1,5-bis-(1-pyrene)-2,4-pentadien-3-one, m.p 279-281°C.

1,5-bis-(1-pyrene)-3-pentanone

Zinc dust, 22 g, was added in small batches to a solution of 3.5 g 1,5-bis-(1-pyrene)-2,4-pentadien-3-one in refluxing acetic acid. The orange color dissipated to yield a yellow solution. The solution was decanted into water and extracted with methylene chloride; the zinc was washed with $CH_2Cl_2$/water. The combined organic layers were dried over $Na_2SO_4$. Evaporation of solvent yielded 2.71 g crude product. Thin layer chromatography of 2.4 g crude product from the reduction on silica gel (Kieselgel, 70-230 mesh) using toluene as eluent produced five bands; the fourth, with $R_f$=0.25, contained the desired product by NMR. After drying, it may be used in Example 7, above.

While this invention has been described with respect to various specific examples and embodiments, it is to be understood that the invention is not limited thereto and that it can be variously practiced within the scope of the following claims.

**Claims**

1. A sensor for measuring the concentration of an analyte in a medium comprising:

   - a sensing element including a matrix material (20;120) which is permeable to said analyte in said medium and one or more monomeric indicator components in enforced association, wherein
   - each of said one or more monomeric indicator components

     - is positioned or oriented in physical and/or molecular enforcement to promote or facilitate the formation and/or maintenance of a channel or path for excimer component or mixed excimer component formation which (a) is kinetically dominant relative to free diffusion, and/or (b) is kinetically competitive with the decay of the excimer component or the mixed excimer component in the absence of the analyte, and
     - is capable of providing a first emitted signal of a given wavelength in response to being exposed to a first excitation signal,

   - said sensing element being capable of providing a second emitted signal having a longer wavelength than said first emitted signal or signals in response to being exposed to a second excitation signal, said second emitted signal being provided by an excimer component or a mixed excimer component produced in said sensing element from said monomeric indicator component or components and being dependent on the concentration of said analyte in said medium to a greater extent than said first emitted signal or signals,

     - wherein an excimer component refers to an excited state species derived from a molecular or sub-molecular interaction of two or more monomeric indicator components which have the same indicator structure, and
     - wherein a mixed excimer component refers to an excited state species other than an excimer component derived from a molecular or sub-molecular interaction of two or more monomeric indicator components one or more of which is a donor component and one or more of which is an acceptor component and which have sufficiently similar electronic energy levels, ionization potentials and electron affinities to satisfy the following relationship:

$$E_D^{ox} - E_A^{red} > h\nu(hex) + 0.25eV$$

     where $E_D^{ox}$ is the ground state oxidation potential of the donor component, $E_A^{red}$ is the ground state reduction potential of the acceptor component, each measured relative to the same standard, $h\nu$(hex) is the maximum energy in eV (electron volts) of the excited state species measured in n-hexane, and the donor component and acceptor component are assigned so that the absolute value of $E_D^{ox} - E_A^{red}$ is minimized,

   - an excitation assembly (14;114) positioned and adapted to provide said first and second excitation signals to said sensing element;
   - a detector assembly (16;116) positioned and adapted to detect said first emitted signal or signals and said second emitted signal; and
   - a processor assembly (17;117) positioned and adapted to analyze said first emitted signal or signals and said second emitted signal for determining the concentration of said analyte in said medium.

2. The sensor according to claim 1 characterized in that said first emitted signal or at least one of said first emitted signals is dependent on the concentration of said analyte in said medium and said second emitted signal is dynamically quenchable by said analyte in said medium.

3. The sensor according to claim 1 or 2 characterized in that said processor assembly (17;117) is adapted to determine the ratio of said second emitted signal to said first emitted signal or to one of said first emitted signals, said ratio being dependent on the concentration of said analyte in said medium.

4. The sensor according to any one of claims 1 to 3 characterized in that said first emitted signal or at least one of said first emitted signals and said second emitted signal are modulated and said processor assembly (17;117) is adapted to determine at least one of

   - the extent of the phase shift between and

- the ratio of demodulation factors of said modulated first emitted signal or at least one of said modulated first emitted signals and said modulated second emitted signal, at least one of said extent and said ratio being dependent on the concentration of said analyte in said medium.

**5.** The sensor according to any one of claims 1 to 4 characterized in that said matrix material (20;120) is solid and said monomeric indicator component or components are covalently bonded to said solid matrix material (20;120).

**6.** The sensor according to any one of claims 1 to 5 characterized in that said first emitted signal or signals and said second emitted signal are the result of fluorescing.

**7.** The sensor according to any one of claims 1 to 5 characterized in that said monomeric indicator component or components are selected from the group consisting of polynuclear aromatic compounds, derivatives of polynuclear aromatic compounds and mixtures thereof.

**8.** The sensor according to any one of claims 1 to 7 characterized in that said second emitted signal is provided by said excimer component and said monomeric indicator component is selected from the group consisting of benzo [ghi]perylene, derivatives of benzo[ghi]perylene and mixtures thereof.

**9.** The sensor according to any one of claims 1 to 8 characterized in that said first and second excitation signals each has substantially the same wavelength.

**10.** The sensor according to any one of claims 1 to 9 wherein said analyte is oxygen.

**11.** The sensor according to any one of claims 1 to 10 characterized in that said monomeric indicator components in enforced association are selected from the group consisting of intramolecular excimer component or mixed excimer component forming molecules dispersed in a matrix material, intramolecular excimer component or mixed excimer component forming molecules covalently attached to a matrix material, monomeric indicator component aggregates covalently attached to a matrix material or adsorbed to the surface of a matrix material and intramolecular excimer component or mixed excimer component forming molecules covalently attached, adsorbed or otherwise attached to the surface of a matrix material.

**12.** The sensor according to any one of claims 1 to 11 characterized in that said monomeric components are linked by less than 20 atoms.

**13.** The sensor according to any one of claims 1 to 12 characterized in that said monomeric components are linked by between 2 and 7 carbon atoms.

**14.** A method for sensing the concentration of an analyte in a medium comprising:

- exposing a sensing element to said medium, said sensing element including a matrix material (20; 120) which is permeable to said analyte in said medium and one or more monomeric indicator components in enforced association, wherein
- each of said one or more monomeric indicator components
  - is positioned or oriented in physical and/or molecular enforcement to promote or facilitate the formation and/or maintenance of a channel or path for excimer component or mixed excimer component formation which (a) is kinetically dominant relative to free diffusion, and/or (b) is kinetically competitive with the decay of the excimer component or the mixed excimer component in the absence of the analyte, and
  - is capable of providing a first emitted signal of a given wavelength in response to being exposed to a first excitation signal,
- said sensing element being capable of providing a second emitted signal having a longer wavelength than said first emitted signal or signals in response to being exposed to a second excitation signal, said second emitted signal being provided by an excimer component or a mixed excimer component produced in said sensing element from said monomeric indicator component or components and being dependent on the concentration of said analyte in said medium to a greater extent than said first emitted signal or signals,
  - wherein an excimer component refers to an excited state species derived from a molecular or sub-molecular interaction of two or more monomeric indicator components which have the same indicator structure, and

- wherein a mixed excimer component refers to an excited state species other than an excimer component derived from a molecular or sub-molecular interaction of two or more monomeric indicator components one or more of which is a donor component and one or more of which is an acceptor component and which have sufficiently similar electronic energy levels, ionization potentials and electron affinities to satisfy the following relationship:

$$E_D^{ox} - E_A^{red} > h\nu(hex) + 0.25eV$$

where $E_D^{ox}$ is the ground state oxidation potential of the donor component, EredA is the ground state reduction potential of the acceptor component, each measured relative to the same standard, hν(hex) is the maximum energy in eV (electron volts) of the excited state species measured in n-hexane, and the donor component and acceptor component are assigned so that the absolute value of $E_D^{ox} - E_A^{red}$ is minimized,

- causing said sensing element to provide said first emitted signal or signals and said emitted second signal; and
- analyzing said first emitted signal or signals and said second emitted signal for determining the concentration of said analyte in said medium.

**15.** The method according to claim 14 characterized in that said analyzing step includes determining the ratio of said second emitted signal to said emitted first signal or at least one of said first emitted signals, said ratio being dependent on the concentration of said analyte in said medium.

**16.** The method according to claim 14 or 15 characterized in that said first emitted signal or at least one of said first emitted signals and said second emitted signal are modulated, and at least one of

- the extent of the phase shift between and
- the ratio of demodulation factors of said modulated first emitted signal or at least one of said modulated first emitted signals and said modulated second emitted signal is dependent on the concentration of said analyte in said medium.

**17.** The method according to any one of claims 14 to 16 wherein said medium is blood.

**18.** The method according to any one of claims 14 to 17 characterized in that said at least one first signal and said second signal are the result of fluorescing.

**19.** The method according to any one of claims 14 to 18 wherein said monomeric indicator components in enforced association are selected from the group consisting of intramolecular excimer component or mixed excimer component forming molecules dispersed in a matrix material, intramolecular excimer component or mixed excimer component forming molecules covalently attached to a matrix material, monomeric indicator component aggregates covalently attached to a matrix material or adsorbed to the surface of a matrix material and intramolecular excimer component or mixed excimer component forming molecules covalently attached, adsorbed or otherwise attached to the surface of a matrix material.

**20.** A composition useful for measuring the concentration of an analyte in a medium comprising:

- a solid matrix material (20;120) which is permeable to said analyte in said medium; and
- an indicator component in said matrix material (20;120) and including a first species capable of providing a first emitted signal of a first wavelength in response to being exposed to a first excitation signal, and a second species capable of providing a second emitted signal of a second wavelength in response to being exposed to a second excitation signal, said first species being covalently bonded to said second species, provided that when said indicator component is covalently bonded to said solid matrix material, this is done through a single covalent linkage, said composition being capable of providing a third emitted signal in response to being exposed to a third excitation signal, said third emitted signal being provided by an excimer component or a mixed excimer component produced from said first species and said second species, said third emitted signal being dependent on the concentration of said analyte in said medium,

- wherein an excimer component refers to an excited state species derived from a molecular or sub-molecular interaction of two or more monomeric indicator components which have the same indicator structure, and
- wherein a mixed excimer component refers to an excited state species other than an excimer component derived from a molecular or sub-molecular interaction of two or more, preferably two, monomeric indicator components one or more, preferably one, of which is a donor component and one or more, preferably one, of which is an acceptor component and which have sufficiently similar electronic energy levels, ionization potentials and electron affinities to satisfy the following relationship:

$$E_D^{ox} - E_A^{red} > h\nu(hex) + 0.25eV$$

where $E_D^{ox}$ is the ground state oxidation potential of the donor component, $E_A^{red}$ is the ground state reduction potential of the acceptor component, each measured relative to the same standard, $h\nu$(hex) is the maximum energy in eV (electron volts) of the excited state species measured in n-hexane, and the donor component and acceptor component are assigned so that the absolute value of $E_D^{ox} - E_A^{red}$ is minimized.

**21.** A composition useful for measuring the concentration of an analyte in a medium comprising:

- a solid matrix material (20;120) which is permeable to said analyte in said medium, and
- one or more monomeric indicator components in enforced association, wherein,
- each of said one or more monomeric indicator components
  - is positioned or oriented in physical and/or molecular enforcement to promote or facilitate the formation and/or maintenance of a channel or path for excimer component or mixed excimer component formation which (a) is kinetically dominant relative to free diffusion, and/or (b) is kinetically competitive with the decay of the excimer component or the mixed excimer component in the absence of the analyte, and
  - is capable of providing a first emitted signal of a given wavelength in response to being exposed to a first excitation signal,
  - said composition further comprising, when exposed to said excitation signal, an excimer component or a mixed excimer component produced from said monomeric indicator components,
  - wherein an excimer component refers to an excited state species derived from a molecular or sub-molecular interaction of two or more monomeric indicator components which have the same indicator structure, and
  - wherein a mixed excimer component refers to an excited state species other than an excimer component derived from a molecular or sub-molecular interaction of two or more, preferably two, monomeric indicator components one or more, preferably one, of which is a donor component and one or more, preferably one, of which is an acceptor component and which have sufficiently similar electronic energy levels, ionization potentials and electron affinities to satisfy the following relationship:

$$E_D^{ox} - E_A^{red} > h\nu(hex) + 0.25eV$$

where $E_D^{ox}$ is the ground state oxidation potential of the donor component, $E_A^{red}$ is the ground state reduction potential of the acceptor component, each measured relative to the same standard, $h\nu$(hex) is the maximum energy in eV (electron volts) of the excited state species measured in n-hexane, and the donor component and acceptor component are assigned so that the absolute value of $E_D^{ox} - E_A^{red}$ is minimized,

- said excimer component or mixed excimer component providing a second emitted signal, said second signal being dependent on the concentration of said analyte in said medium to a greater extent than said first emitted signal or signals, and said second emitted signal being different and distinct from said first emitted signal or signals.

**Patentansprüche**

**1.** Sensor zum Messen der Konzentration eines Analyten in einem Medium, mit:

- inem Sensorelement mit einem Matrixmaterial (20; 120), das für den Analyten in dem Medium durchlässig ist, und einer oder mehreren verschiedenen Monomerindikatorkomponenten in Zwangsassoziation, bei welchem

- jede der einen oder mehreren Monomerindikatorkomponenten

  - physikalisch und/oder molekular derart zwangspositioniert oder -ausgerichtet ist, daß sie die Bildung und/ oder Erhaltung eines Kanals oder Weges zur Bildung von Excimer-Komponenten oder gemischten Excimer-Komponenten fördert oder erleichtert, die (a) im Vergleich zu der freien Verteilung kinetisch dominant ist und/oder (b) mit dem Verfall der Excimer-Komponente oder der gemischten Excimer-Komponente in Abwesenheit des Analyten kinetisch kompetitiv ist, und

  - geeignet ist, als Reaktion darauf, daß sie einem ersten Anregungssignal ausgesetzt ist, ein erstes Emissionssignal einer gegebenen Wellenlänge zu liefern,

- wobei das Sensorelement geeignet ist, ein zweites Emissionssignal mit einer längeren Wellenlänge als das erste Emissionssignal oder die ersten Emissionssignale als Reaktion darauf, daß es einem zweiten Anregungssignal ausgesetzt ist, zu liefern. wobei das zweite Emissionssignal von einer Excimer-Komponente oder einer gemischten Excimer-Komponente geliefert wird, die in dem Sensorelement aus der Monomerindikatorkomponente oder den Monomerindikatorkomponenten erzeugt wird, und die von der Konzentration des Analyten in dem Medium in größerem Ausmaß abhängig ist als das erste Emissionssignal oder die ersten Emissionssignale,

- bei welchem eine Excimer-Komponente sich auf eine Spezies in einem Anregungszustand bezieht. die durch eine molekulare oder submolekulare Interaktion von zwei oder mehr verschiedenen Monomerindikatorkomponenten mit derselben Indikatorstruktur erhalten wird, und

- bei welchem eine gemischte Excimer-Komponente sich auf eine andere Spezies in einem Anregungszustand bezieht als eine Excimer-Komponente, die aus einer molekularen oder submolekulaten Interaktion von zwei oder mehr verschiedenen Monomerindikatorkomponenten erhalten wird, von denen eine oder mehr eine Donatorkomponente ist und von denen eine oder mehr eine Akzeptorkomponente ist, und die ausreichend ähnliche elektronische Energiezustände, Ionisationspotentiale und Elektronenaffinitäten aufweisen, um die folgende Beziehung zu erfüllen:

$$E_D^{ox} - E_A^{red} > hv(hex) + 0{,}25\,eV$$

  wobei $E_D^{ox}$ das Grundzustandsoxidationspotential der Donatorkomponente ist, $E_A^{red}$ das Grundzustandsreduktionspotential der Akzeptorkomponente ist, die jeweils im Vergleich zu demselben Standard gemessen sind, *hv(hex)* die Maximalenergie in eV (Elektronenvolt) der in n-Hexan gemessenen Spezies in einem Anregungszustand ist, und die Donatorkomponente und die Akzeptorkomponente derart zugeordnet sind, daß der absolute Wert von $E_D^{ox} - E_A^{red}$ minimiert wird,

- einer Anregungsanordnung (14; 114), die derart angeordnet und geeignet ist, daß sie die ersten und zweiten Anregungssignale an das Sensorelement liefert;

- einer Detektoranordnung (16; 116), die derart angeordnet und geeignet ist, daß sie das erste Emissionssignal oder die ersten Emissionssignale und das zweite Emissionssignal detektiert; und

- einer Prozessoranordnung (17; 117), die derart angeordnet und geeignet ist, daß sie das erste Emissionssignal oder die ersten Emissionssignale und das zweite Emissionssignal zum Bestimmen der Konzentration des Analyten in dem Medium analysiert.

**2.** Sensor nach Anspruch 1, dadurch gekennzeichnet, daß das erste Emissionssignal oder wenigstens eines der ersten Emissionssignale von der Konzentration des Analyten in dem Medium abhängig ist und das zweite Emissionssignal durch den Analyten in dem Medium dynamisch löschbar ist.

**3.** Sensor nach Anspruch 1 oder 2, dadurch gekennzeichnet, daß die Prozessoranordnung (17; 117) geeignet ist, das Verhältnis des zweiten Emissionssignals zu dem ersten Emissionssignal oder zu den ersten Emissionssignalen zu bestimmen, wobei das Verhältnis von der Konzentration des Analyten in dem Medium abhängig ist.

**4.** Sensor nach einem der Ansprüche 1 bis 3, dadurch gekennzeichnet, daß das erste Emissionssignal oder wenigstens eines der ersten Emissionssignale und das zweite Emissionssignal moduliert sind und die Prozessoranordnung (17; 117) geeignet ist, wenigstens eines der folgenden Merkmale zu bestimmen:

- das Ausmaß der Phasenverschiebung zwischen dem modulierten ersten Emissionssignal oder wenigstens einem der ersten Emissionssignale und dem modulierten zweiten Emissionssignal und

- das Verhältnis der relativen Demodulationsfaktoren zwischen dem modulierten ersten Emissionssignal oder wenigstens einem der ersten Emissionssignale und dem modulierten zweiten Emissionssignal,

wobei wenigstens ein Merkmal aus der Gruppe des Ausmaßes und des Verhältnisses von der Konzentration des Analyten in dem Medium abhängig ist.

**5.** Sensor nach einem der Ansprüche 1 bis 4, dadurch gekennzeichnet, daß das Matrixmaterial (20; 120) fest ist und die Monomerindikatorkomponente oder die Monomerindikatorkomponenten an dem festen Matrixmaterial (20; 120) kovalent gebunden sind.

**6.** Sensor nach einem der Ansprüche 1 bis 5, dadurch gekennzeichnet, daß das erste Emissionssignal oder die ersten Emissionssignale und das zweite Emissionssignal das Ergebnis von Fluoreszenz sind.

**7.** Sensor nach einem der Ansprüche 1 bis 5, dadurch gekennzeichnet, daß die Monomerindikatorkomponente oder die Monomerindikatorkomponenten aus der Gruppe ausgewählt sind, die polynukleare aromatische Verbindungen, Derivate polynuklearer aromatischer Verbindungen und Mischungen hieraus aufweist.

**8.** Sensor nach einem der Ansprüche 1 bis 7, dadurch gekennzeichnet, daß das zweite Emissionssignal durch die Excimer-Komponente geliefert wird und die Monomerindikatorkomponente aus der Gruppe ausgewählt ist, die Benzo[ghi]perylen, Derivate von Benzo[ghi]perylen und Mischungen hieraus aufweist.

**9.** Sensor nach einem der Ansprüche 1 bis 8, dadurch gekennzeichnet, daß die ersten und zweiten Anregungssignale jeweils im wesentlichen dieselbe Wellenlänge aufweisen.

**10.** Sensor nach einem der Ansprüche 1 bis 9, bei welchem der Analyt Sauerstoff ist.

**11.** Sensor nach einem der Ansprüche 1 bis 10, dadurch gekennzeichnet, daß die Monomerindikatorkomponenten in Zwangsassoziation aus der Gruppe ausgewählt sind, die in einem Matrixmaterial fein verteilten, eine intramolekulare Excimer-Komponente oder gemischte Excimer-Komponente bildenden Molekülen, kovalent an einem Matrixmaterial gebundenen, eine intramolekulare Excimer-Komponente oder gemischte Excimer-Komponente bildenden Molekülen, kovalent an einem Matrixmaterial gebundenen oder an der Oberfläche eines Matrixmaterials adsorbierten Monomerindikatorkomponentaggregaten und kovalent an der Oberfläche eines Matrixmaterials gebundenen, daran adsorbierten oder auf andere Weise angebrachten, eine intramolekulare Excimer-Komponente oder gemischte Excimer-Komponente bildenden Molekülen aufweist.

**12.** Sensor nach einem der Ansprüche 1 bis 11, dadurch gekennzeichnet, daß die Monomerkomponenten durch weniger als 20 Atome verbunden sind.

**13.** Sensor nach einem der Ansprüche 1 bis 12, dadurch gekennzeichnet, daß die Monomerkomponenten durch zwischen 2 und 7 Kohlenstoffatome verbunden sind.

**14.** Verfahren zum Erfassen der Konzentration eines Analyten in einem Medium, mit den Schritten:

- Aussetzen eines Sensorelements dem Medium, wobei das Sensorelement ein Matrixmaterial (20; 120), das für den Analyten in dem Medium durchlässig ist, und eine oder mehr verschiedene Monomerindikatorkomponenten in Zwangsassoziation aufweist, bei welchem

- jede der einen oder mehreren Monomerindikatorkomponenten

- physikalisch und/oder molekular derart zwangspositioniert oder -ausgerichtet ist, daß sie die Bildung und/oder Erhaltung eines Kanals oder Weges zur Bildung einer Excimer-Komponente oder einer gemischten Excimer-Komponente fördert oder erleichtert, die (a) im Vergleich zu der freien Verteilung kinetisch dominant ist und/oder (b) mit dem Verfall der Excimer-Komponente oder der gemischten Excimer-Komponente in Abwesenheit des Analyten kinetisch kompetitiv ist, und

- geeignet ist, als Reaktion darauf, daß sie einem ersten Anregungssignal ausgesetzt ist, ein erstes Emissionssignal einer gegebenen Wellenlänge zu liefern,

- wobei das Sensorelement geeignet ist, ein zweites Emissionssignal mit einer längeren Wellenlänge als das erste Emissionssignal oder die ersten Emissionssignale als Reaktion darauf, daß es einem zweiten Anregungssignal ausgesetzt ist, zu liefern, wobei das zweite Emissionssignal von einer Excimer-Komponente oder einer gemischten Excimer-Komponente geliefert wird, die in dem Sensorelement aus der Monomerindikatorkomponente oder den Monomerindikatorkomponenten erzeugt wird, und die von der Konzentration des Analyten in dem Medium in größerem Ausmaß abhängig ist als das erste Emissionssignal oder die ersten Emissionssignale,

- bei welchem eine Excimer-Komponente sich auf eine Spezies in einem Anregungszustand bezieht, die durch eine molekulare oder submolekulare Interaktion von zwei oder mehr verschiedenen Monomerindikatorkomponenten erhalten wird, die dieselbe Indikatorstruktur aufweisen, und

- bei welchem eine gemischte Excimer-Komponente sich auf eine andere Spezies in einem Anregungszustand bezieht als eine Excimer-Komponente, die aus einer molekularen oder submolekulaten Interaktion von zwci oder mehr verschiedenen Monomerindikatorkomponenten erhalten wird, von denen eine oder mehr eine Donatorkomponente ist und von denen eine oder mehr eine Akzeptorkomponente ist, und die ausreichend ähnliche elektronische Energiezustände, Ionisationspotentiale und Elektronenaffinitäten aufweisen, um die folgende Beziehung zu erfüllen:

$$E_D^{ox} - E_A^{red} > hv(hex) + 0{,}25\,eV$$

  wobei $E_D^{ox}$ das Grundzustandsoxidationspotential der Donatorkomponente ist, $E_A^{red}$ das Grundzustandsreduktionspotential der Akzeptorkomponente ist, die jeweils im Vergleich zu demselben Standard gemessen sind, *hv(hex)* die Maximalenergie in eV (Elektronenvolt) der in n-Hexan gemessenen Spezies in einem Anregungszustand ist, und die Donatorkomponente und die Akzeptorkomponente derart zugeordnet sind, daß der absolute Wert von $E_D^{ox} - E_A^{red}$ minimiert wird,

- Bewirken, daß das Sensorelement das erste Emissionssignal oder die ersten Emissionssignale und das zweite Emissionssignal liefert; und

- Analysieren des ersten Emissionssignals oder der ersten Emissionssignale und des zweiten Emissionssignals zum Bestimmen der Konzentration des Analyten in dem Medium.

**15.** Verfahren nach Anspruch 14, dadurch gekennzeichnet, daß der Schritt des Analysierens es umfaßt, das Verhältnis des zweiten Emissionssignals zu dem ersten Emissionssignal oder wenigstens einem der ersten Emissionssignale zu bestimmen, wobei das Verhältnis von der Konzentration des Analyten in dem Medium abhängig ist.

**16.** Verfahren nach Anspruch 14 oder 15, dadurch gekennzeichnet, daß das erste Emissionssignal oder wenigstens eines der ersten Emissionssignale und das zweite Emissionssignal moduliert sind, und das Ausmaß der Phasenverschiebung und/oder das Verhältnis der relativen Demodulationsfaktoren zwischen dem modulierten ersten Emissionssignal oder wenigstens einem der ersten Emissionssignale und dem modulierten zweiten Emissionssignal von der Konzentration des Analyten in dem Medium abhängig ist.

**17.** Verfahren nach einem der Ansprüche 14 bis 16, bei welchem das Medium Blut ist.

**18.** Verfahren nach einem der Ansprüche 14 bis 17, dadurch gekennzeichnet, daß wenigstens eines der ersten Signale und das zweite Signal das Ergebnis von Fluoreszenz sind.

**19.** Verfahren nach einem der Ansprüche 14 bis 18, bei welchem die Monomerindikatorkomponenten in Zwangsassoziation aus der Gruppe ausgewählt sind, die aus in einem Matrixmaterial fein verteilten, eine intramolekulare Excimer-Komponente oder gemischte Excimer-Komponente bildenden Molekülen, kovalent an einem Matrixmaterial gebundenen, eine intramolekulare Excimer-Komponente oder gemischte Excimer-Komponente bildenden Molekülen, kovalent an einem Matrixmaterial gebundenen oder an der Oberfläche eines Matrixmaterials adsorbierten Monomerindikatorkomponentaggregaten, oder kovalent gebundenen, adsorbierten oder auf andere Weise an der Oberfläche eines Matrixmaterials angelagerten, eine intramolekulare Excimer-Komponente oder gemischte Excimer-Komponente bildenden Molekülen besteht.

**20.** Zuammensetzung zum Messen der Konzentration eines Analyten in einem Medium, mit:

- einem festen Matrixmaterial (20; 120), das für den Analyten in dem Medium durchlässig ist; und

- einer Indikatorkomponente in dem Matrixmaterial (20; 120) und mit einer ersten Spezies, die geeignet ist, als Reaktion auf das Aussetzen eines ersten Anregungssignals ein erstes Emissionssignal einer ersten Wellenlänge zu erzeugen, und einer zweiten Spezies, die geeignet ist, als Reaktion auf das Aussetzen eines zweiten Anregungssignals ein zweites Emissionssignal einer zweiten Wellenlänge zu erzeugen, wobei die erste Spezies kovalent an der zweiten Spezies gebunden ist, vorausgesetzt, daß, wenn die Indikatorkomponente kovalent an dem festen Matrixmaterial gebunden ist, dies durch eine einzelne kovalente Bindung geschieht, wobei die Zusammensetzung geeignet ist, als Reaktion auf das Aussetzen eines dritten Anregungssignals ein drittes Emissionssignal zu liefern, wobei das dritte Emissionssignal durch eine Excimer-Komponente oder eine aus der ersten Spezies und der zweiten Spezies erzeugte gemischte Excimer-Komponente geliefert wird, wobei das dritte Emissionssignal von der Konzentration des Analyten in dem Medium abhängig ist,

- bei welchem eine Excimer-Komponente sich auf eine Spezies in einem Anregungszustand bezieht, die durch eine molekulare oder submolekulare Interaktion von zwei oder mehr verschiedenen Monomerindikatorkomponenten mit derselben Indikatorstruktur erhalten wird, und

- bei welchem eine gemischte Excimer-Komponente sich auf eine andere Spezies in einem Anregungszustand bezieht als eine Excimer-Komponente, die aus einer molekularen oder submolekularen Interaktion von zwei oder mehr, vorzugsweise zwei, verschiedenen Monomerindikatorkomponenten erhalten wird, von denen eine oder mehr, vorzugsweise eine, eine Donatorkomponente ist und von denen eine oder mehr, vorzugsweise eine, eine Akzeptorkomponente ist, und die ausreichend ähnliche elektronische Energiezustände, Ionisationspotentiale und Elektronenaffinitäten aufweisen, um die folgende Beziehung zu erfüllen:

$$E_D^{ox} - E_A^{red} > hv(hex) + 0{,}25\,eV$$

- wobei $E_D^{ox}$ das Grundzustandsoxidationspotential der Donatorkomponente ist, $E_A^{red}$ das Grundzustandsreduktionspotential der Akzeptorkomponente ist, die jeweils im Vergleich zu demselben Standard gemessen sind, *hv(hex)* die Maximalenergie in eV (Elektronenvolt) der in n-Hexan gemessenen Spezies in einem Anregungszustand ist, und die Donatorkomponente und die Akzeptorkomponente derart zugeordnet sind, daß der absolute Wert von $E_D^{ox} - E_A^{red}$ minimiert wird.

**21.** Zuammensetzung, die zum Messen der Konzentration eines Analyten in einem Medium nützlich ist, mit:

- einem festen Matrixmaterial (20; 120), das für den Analyten in dem Medium durchlässig ist; und

- einer oder mehr Monomerindikatorkomponenten in Zwangsassoziation, bei welchem
  - jede der einen oder mehreren Monomerindikatorkomponenten

  - physikalisch und/oder molekular derart zwangspositioniert oder -ausgerichtet ist, daß sie die Bildung und/oder Erhaltung eines Kanals oder Weges zur Bildung von Excimer-Komponenten oder gemischten Excimer-Komponenten fördert oder erleichtert, die (a) im Vergleich zu der freien Verteilung kinetisch dominant ist und/oder (b) mit dem Verfall der Excimer-Komponente oder der gemischten Excimer-Komponente in Abwesenheit des Analyten kinetisch kompetitiv ist, und

  - geeignet ist, als Reaktion darauf, daß sie einem ersten Anregungssignal ausgesetzt ist, ein erstes Emissionssignal einer gegebenen Wellenlänge zu liefern,

- wobei die Zusammensetzung, wenn sie dem Anregungssignal ausgesetzt ist, ferner eine Excimer-Komponente oder eine gemischte Excimer-Komponente aufweist, die aus den Monomerindikatorkomponenten erzeugt wird,

- bei welchem eine Excimer-Komponente sich auf eine Spezies in einem Anregungszustand bezieht, die durch eine molekulare oder submolekulare Interaktion von zwei oder mehr Monomerindikatorkomponenten mit derselben Indikatorstruktur erhalten wird, und

- bei welchem eine gemischte Excimer-Komponente sich auf eine andere Spezies in einem Anregungszustand bezieht als eine Excimer-Komponente, die aus einer molekularen oder submolekulaten Interaktion von zwei oder mehr, vorzugsweise zwei, verschiedenen Monomerindikatorkomponenten erhalten wird, von denen eine oder mehr, vorzugsweise eine, eine Donatorkomponente ist und von denen eine oder mehr, vorzugsweise eine, eine Akzeptorkomponente ist, und die ausreichend ähnliche elektronische Energiezustände, Ionisationspotentiale und Elektronenaffinitäten aufweisen, um die folgende Beziehung zu erfüllen:

$$E_D^{ox} - E_A^{red} > hv(hex) + 0{,}25\,eV$$

- wobei $E_D^{ox}$ das Grundzustandsoxidationspotential der Donatorkomponente ist, $E_A^{red}$ das Grundzustandsreduktionspotential der Akzeptorkomponente ist, die jeweils im Vergleich zu demselben Standard gemessen sind, *hv(hex)* die Maximalenergie in eV (Elektronenvolt) der in n-Hexan gemessenen Spezies in einem Anregungszustand ist, und die Donatorkomponente und die Akzeptorkomponente derart zugeordnet sind, daß der absolute Wert von $E_D^{ox} - E_A^{red}$ minimiert wird,

- wobei die Excimer-Komponente oder die gemischte Excimer-Komponente ein zweites Emissionssignal liefert, das von der Konzentration des Analyten in dem Medium in einem größeren Ausmaß als das erste Emissionssignal oder die ersten Emissionssignale abhängig ist und das zweite Emissionssignal von dem ersten Emissionssignal oder den ersten Emissionssignalen verschieden und unterschiedlich ist.

## Revendications

**1.** Capteur pour mesurer la concentration d'un analyte dans un milieu, comprenant :

- un élément capteur comprenant un matériau de matrice (20 ; 120), qui est perméable audit analyte dans ledit milieu, et un ou plusieurs composants indicateurs monomères, en association contrainte, où
- chacun desdits un ou plusieurs composants indicateurs monomères

    - est positionné ou orienté selon une contrainte physique et/ou moléculaire pour favoriser ou faciliter la formation et/ou le maintien d'un canal ou d'un chemin pour la formation d'un composant excimère ou d'un composant excimère mixte, qui (a) est cinétiquement dominante par rapport à la diffusion libre, et/ou (b) est cinétiquement compétitive avec la désintégration du composant excimère ou du composant excimère mixte en l'absence de l'analyte, et
    - est capable de fournir un premier signal émis, ayant une longueur d'onde donnée, en réponse à son exposition à un premier signal d'excitation,

- ledit élément capteur étant capable de fournir un deuxième signal émis, ayant une longueur d'onde plus grande que celle dudit ou desdits premiers signaux émis, en réponse à une exposition à un deuxième signal d'excitation, ledit deuxième signal émis étant fourni par un composant excimère ou par un composant excimère mixte produit dans ledit élément capteur à partir dudit ou desdits composants indicateurs monomères, et dépendant de la concentration dudit analyte dans ledit milieu dans une mesure plus grande que ledit ou lesdits premiers signaux émis,

    - où un composant excimère désigne une espèce dans un état excité, qui dérive d'une interaction moléculaire ou sous-moléculaire de deux composants indicateurs monomères ou plus, qui ont la même structure

d'indicateur, et

- où un composant excimère mixte désigne une espèce dans un état excité, autre qu'un composant excimère dérivant d'une interaction moléculaire ou sous-moléculaire de deux composants indicateurs monomères ou plus, dont l'un ou plusieurs est un composant donneur, et dont l'un ou plus est un composant accepteur, et qui ont des niveaux d'énergie électronique, des potentiels d'ionisation et des affinités d'électrons suffisamment analogues pour satisfaire à la relation suivante :

$$E_D^{ox} - E_A^{red} > h\nu(hex) + 0{,}25eV$$

où $E_D^{ox}$ est le potentiel d'oxydation de base du composé donneur, $E_A^{red}$ est le potentiel de réduction de base du composant accepteur, chacun étant mesuré par rapport au même étalon, hν(hex) est l'énergie maximale, en eV (électronvolt) de l'espèce dans un état excité, mesurée dans le n-hexane, et le composant donneur et le composant accepteur sont affectés de façon à minimiser la valeur absolue de $E_D^{ox} - E_A^{red}$,

- un ensemble d'excitation (14; 114), positionné et adapté de façon à mettre à la disposition dudit élément capteur lesdits premier et deuxième signaux d'excitation ;
- un ensemble détecteur (16 ; 116) positionné et adapté de façon à détecter ledit ou lesdits premiers signaux émis et ledit deuxième signal émis ; et
- un ensemble processeur (17 ; 117) positionné et adapté de façon à analyser ledit ou lesdits premiers signaux émis et ledit deuxième signal émis, pour déterminer la concentration dudit analyte dans ledit milieu.

**2.** Capteur selon la revendication 1, caractérisé en ce que ledit premier signal émis, ou au moins l'un desdits premiers signaux émis, dépend de la concentration dudit analyte dans ledit milieu, et ledit deuxième signal émis peut subir une interruption dynamique brutale par ledit analyte dans ledit milieu.

**3.** Capteur selon la revendication 1 ou 2, caractérisé en ce que ledit ensemble processeur (17 ; 117) est adapté de façon à déterminer le rapport dudit deuxième signal émis audit premier signal émis ou à l'un desdits premiers signaux émis, ledit rapport dépendant de la concentration dudit analyte dans ledit milieu.

**4.** Capteur selon l'une quelconque des revendications 1 à 3, caractérisé en ce que ledit premier signal émis ou au moins l'une desdits premiers signaux émis et ledit deuxième signal émis sont modulés, et ledit ensemble processeur (17 ; 117) est adapté pour déterminer au moins l'une des paramètres suivants :

- la valeur du déphasage entre ledit premier signal émis modulé ou au moins l'une desdits premiers signaux émis modulés et ledit deuxième signal émis modulé, et
- le rapport entre les facteurs de démodulation dudit premier signal émis modulé ou au moins l'un desdits premiers signaux émis modulés et ledit deuxième signal émis modulé,

au moins la valeur ci-dessus, ou le rapport ci-dessus, dépendant de la concentration dudit analyte dans ledit milieu.

**5.** Capteur selon l'une quelconque des revendications 1 à 4, caractérisé en ce que ledit matériau de matrice (20 ; 120) est solide, et ledit ou lesdits composants indicateurs monomères sont liés par liaison covalente audit matériau de matrice solide (20 ; 120).

**6.** Capteur selon l'une quelconque des revendications 1 à 5, caractérisé en ce que ledit ou lesdits premiers signaux émis et ledit deuxième signal émis sont le résultat d'une fluorescence.

**7.** Capteur selon l'une quelconque des revendications 1 à 5, caractérisé en ce que ledit ou lesdits composants indicateurs monomères sont choisis parmi l'ensemble comprenant les composés aromatiques polynucléaires, les dérivés de composés aromatiques polynucléaires et leurs mélanges.

**8.** Capteur selon l'une quelconque des revendications 1 à 7, caractérisé en ce que ledit deuxième signal émis est fourni par ledit composant excimère, et ledit composant indicateur monomère est choisi parmi l'ensemble comprenant le benzo[ghi]pérylène, les dérivés du benzo[ghi]pérylène et leurs mélanges.

**9.** Capteur selon l'une quelconque des revendications 1 à 8, caractérisé en ce que lesdits premiers et deuxièmes signaux d'excitation ont chacun essentiellement la même longueur d'onde.

**10.** Capteur selon l'une quelconque des revendications 1 à 9, dans lequel ledit analyte est l'oxygène.

**11.** Capteur selon l'une quelconque des revendications 1 à 10, caractérisé en ce que lesdits composants indicateurs monomères en association forcée sont choisis parmi l'ensemble comprenant les molécules formant un composant excimère intramoléculaire ou un composant excimère mixte en dispersion dans un matériau de matrice, les molécules formant un composant excimère intramoléculaire ou un composant excimère mixte, liées par liaison covalente à un matériau de matrice, les agrégats de composants indicateurs monomères liés par liaison covalente à un matériau de matrice ou adsorbés par la surface d'un matériau de matrice, et les molécules formant un composant excimère intramoléculaire ou un composant excimère mixte, liées par liaison covalente à la surface d'un matériau de matrice, adsorbées par cette surface ou autrement fixées à cette surface.

**12.** Capteur selon l'une quelconque des revendications 1 à 11, caractérisé en ce que lesdits composants monomères sont liés par moins de 20 atomes de carbone.

**13.** Capteur selon l'une quelconque des revendications 1 à 12, caractérisé en ce que lesdits composants monomères sont liés par un nombre d'atomes de carbone compris entre 2 et 7.

**14.** Procédé pour capter la concentration d'un analyte dans un milieu, consistant :

- à exposer un élément capteur audit milieu ledit élément capteur comprenant un matériau de matrice (20 ; 120), qui est perméable audit analyte dans ledit milieu, et un ou plusieurs composants indicateurs monomères, en association contrainte, où
- chacun desdits un ou plusieurs composants indicateurs monomères

  - est positionné ou orienté selon une contrainte physique et/ou moléculaire pour favoriser ou faciliter la formation et/ou le maintien d'un canal ou d'un chemin pour la formation d'un composant excimère ou d'un composant excimère mixte, qui (a) est cinétiquement dominante par rapport à la diffusion libre, et/ou (b) est cinétiquement compétitive avec la désintégration du composant excimère ou du composant excimère mixte en l'absence de l'analyte, et
  - est capable de fournir un premier signal émis, ayant une longueur d'onde donnée, en réponse à son exposition à un premier signal d'excitation,

- ledit élément capteur étant capable de fournir un deuxième signal émis, ayant une longueur d'onde plus grande que celle dudit ou desdits premiers signaux émis, en réponse à une exposition à un deuxième signal d'excitation, ledit deuxième signal émis étant fourni par un composant excimère ou par un composant excimère mixte produit dans ledit élément capteur à partir dudit ou desdits composants indicateurs monomères, et dépendant de la concentration dudit analyte dans ledit milieu dans une mesure plus grande que ledit ou lesdits premiers signaux émis,

  - où un composant excimère désigne une espèce dans un état excité, qui dérive d'une interaction moléculaire ou sous-moléculaire de deux composants indicateurs monomères ou plus, qui ont la même structure d'indicateur, et
  - où un composant excimère mixte désigne une espèce dans un état excité, autre qu'un composant excimère dérivant d'une interaction moléculaire ou sous-moléculaire de deux composants indicateurs monomères ou plus, dont l'un ou plusieurs est un composant donneur, et dont l'un ou plus est un composant accepteur, et qui ont des niveaux d'énergie électronique, des potentiels d'ionisation et des affinités d'électrons suffisamment analogues pour satisfaire à la relation suivante :

$$E_D^{ox} - E_A^{red} > h\nu(hex) + 0,25eV$$

  où $E_D^{ox}$ est le potentiel d'oxydation de base du composé donneur, $E_A^{red}$ est le potentiel de réduction de base du composant accepteur, chacun étant mesuré par rapport au même étalon, hν(hex) est l'énergie maximale, en eV (électronvolt) de l'espèce dans un état excité, mesurée dans le n-hexane, et le composant donneur et le composant accepteur sont affectés de façon à minimiser la valeur absolue de $E_D^{ox}$- $E_A^{red}$,
  - à amener ledit élément capteur à fournir ledit ou lesdits premiers signaux émis et ledit deuxième signal émis ; et
  - à analyser ledit ou lesdits premiers signaux émis et ledit deuxième signal émis pour déterminer la concentration dudit analyte dans ledit milieu.

**15.** Procédé selon la revendication 14, caractérisé en ce que ladite étape d'analyse consiste à déterminer le rapport dudit deuxième signal émis audit premier signal émis ou à au moins l'une desdits premiers signaux émis, ledit rapport dépendant de la concentration dudit analyte dans ledit milieu.

**16.** Procédé selon la revendication 14 ou 15, caractérisé en ce que ledit premier signal émis ou au moins l'un desdits premiers signaux émis et ledit deuxième signal émis sont modulés, et au moins l'un des paramètres suivants :

- la valeur du déphasage entre ledit premier signal émis modulé ou au moins l'une desdits premiers signaux émis modulés et ledit deuxième signal émis modulé, et
- le rapport entre les facteurs de démodulation dudit premier signal émis modulé ou au moins l'un desdits premiers signaux émis modulés et ledit deuxième signal émis modulé,

dépend de la concentration dudit analyte dans ledit milieu.

**17.** Procédé selon l'une quelconque des revendications 14 à 16, dans lequel ledit milieu est le sang.

**18.** Procédé selon l'une quelconque des revendications 14 à 17, caractérisé en ce que ledit premier signal émis ou au moins l'un desdits premiers signaux émis et ledit deuxième signal sont le résultat d'une fluorescence.

**19.** Procédé selon l'une quelconque des revendications 14 à 18, dans lequel lesdits composants indicateurs monomères en association forcée sont choisis parmi l'ensemble comprenant les molécules formant un composant excimère intramoléculaire ou un composant excimère mixte en dispersion dans un matériau de matrice, les molécules formant un composant excimère intramoléculaire ou un composant excimère mixte, liées par liaison covalente à un matériau de matrice, les agrégats de composants indicateurs monomères liés par liaison covalente à un matériau de matrice ou adsorbés par la surface d'un matériau de matrice, et les molécules formant un composant excimère intramoléculaire ou un composant excimère mixte, liées par liaison covalente à la surface d'un matériau de matrice, adsorbées par cette surface ou autrement fixées à cette surface.

**20.** Composition pouvant être utilisée pour mesurer la concentration d'un analyte dans un milieu, comprenant :

- un matériau de matrice solide (20 ; 120) qui est perméable audit analyte dans ledit milieu ; et
- un composant indicateur dans ledit matériau de matrice (20 ; 120) et comprenant une première espèce capable de fournir un premier signal émis ayant une première longueur d'onde en réponse à une exposition à un premier signal d'excitation, et une deuxième espèce capable de fournir un deuxième signal émis ayant une deuxième longueur d'onde en réponse à une exposition à un deuxième signal d'excitation, ladite première espèce étant liée par liaison covalente à ladite deuxième espèce, du moment que, quand ledit composant indicateur est lié par liaison covalente audit matériau de matrice solide, cette opération est effectuée par une liaison covalente unique, ladite composition étant capable de fournir un troisième signal émis en réponse à une exposition à un troisième signal d'excitation, ledit troisième signal émis étant fourni par un composant excimère ou un composant excimère mixte produit à partir de ladite première espèce et de ladite deuxième espèce, ledit troisième signal émis dépendant de la concentration dudit analyte dans ledit milieu,

  - où un composant excimère désigne une espèce dans un état excité, qui dérive d'une interaction moléculaire ou sous-moléculaire de deux composants indicateurs monomères ou plus, qui ont la même structure d'indicateur, et
  - où un composant excimère mixte désigne une espèce dans un état excité, autre qu'un composant excimère qui dérive d'une interaction moléculaire ou sous-moléculaire de deux ou plus, de préférence deux, composants indicateurs monomères dont l'un ou plus, de préférence l'un, est un composant donneur, et dont l'un ou plus, de préférence l'un, est un composant accepteur, et qui ont des niveaux d'énergie électronique, des potentiels d'ionisation et des affinités d'électrons suffisamment analogues pour satisfaire à la relation suivante :

$$E_D^{ox} - E_A^{red} > h\nu(hex) + 0{,}25eV$$

où $E_D^{ox}$ est le potentiel d'oxydation de base du composé donneur, $E_A^{red}$ est le potentiel de réduction de

base du composant accepteur, chacun étant mesuré par rapport au même étalon, hν(hex) est l'énergie maximale, en eV (électronvolt) de l'espèce dans un état excité, mesurée dans le n-hexane, et le composant donneur et le composant accepteur sont affectés de façon à minimiser la valeur absolue de $E_D^{ox} - E_A^{red}$.

**21.** Composition pouvant être utilisée pour mesurer la concentration d'un analyte dans un milieu, comprenant :

- un matériau de matrice solide (20 ; 120) qui est perméable audit analyte dans ledit milieu, et
- un ou plusieurs composants indicateurs monomères en association forcée, où
- chacun desdits un ou plusieurs composants indicateurs monomères
  - est positionné ou orienté selon une contrainte physique et/ou moléculaire pour favoriser ou faciliter la formation et/ou le maintien d'un canal ou d'un chemin pour la formation d'un composant excimère ou d'un composant excimère mixte, qui (a) est cinétiquement dominante par rapport à la diffusion libre, et/ou (b) est cinétiquement compétitive avec la désintégration du composant excimère ou du composant excimère mixte en l'absence de l'analyte, et
  - est capable de fournir un premier signal émis, ayant une longueur d'onde donnée, en réponse à son exposition à un premier signal d'excitation,
  - ladite composition comprenant en outre, quand elle est exposée audit signal d'excitation, un composant excimère ou un composant excimère mixte produit à partir desdits composants indicateurs monomères,
  - où un composant excimère désigne une espèce dans un état excité, qui dérive d'une interaction moléculaire ou sous-moléculaire de deux composants indicateurs monomères ou plus, qui ont la même structure d'indicateur, et
  - où un composant excimère mixte désigne une espèce dans un état excité, autre qu'un composant excimère qui dérive d'une interaction moléculaire ou sous-moléculaire de deux ou plus, de préférence deux, composants indicateurs monomères dont l'un ou plus, de préférence l'un, est un composant donneur, et dont l'un ou plus, de préférence l'un, est un composant accepteur, et qui ont des niveaux d'énergie électronique, des potentiels d'ionisation et des affinités d'électrons suffisamment analogues pour satisfaire à la relation suivante :

$$E_D^{ox} - E_A^{red} > h\nu(hex) + 0,25eV$$

    où $E_D^{ox}$ est le potentiel d'oxydation de base du composé donneur, $E_A^{red}$ est le potentiel de réduction de base du composant accepteur, chacun étant mesuré par rapport au même étalon, hν(hex) est l'énergie maximale, en eV (électronvolt) de l'espèce dans un état excité, mesurée dans le n-hexane, et le composant donneur et le composant accepteur sont affectés de façon à minimiser la valeur absolue de $E_D^{ox} - E_A^{red}$,

- ledit composant excimère ou composant excimère mixte fournissant un deuxième signal émis, le deuxième signal dépendant de la concentration dudit analyte dans ledit milieu dans une mesure plus grande que ledit ou lesdits premiers signaux émis, et ledit deuxième signal émis étant différent et distinct dudit ou desdits premiers signaux émis.

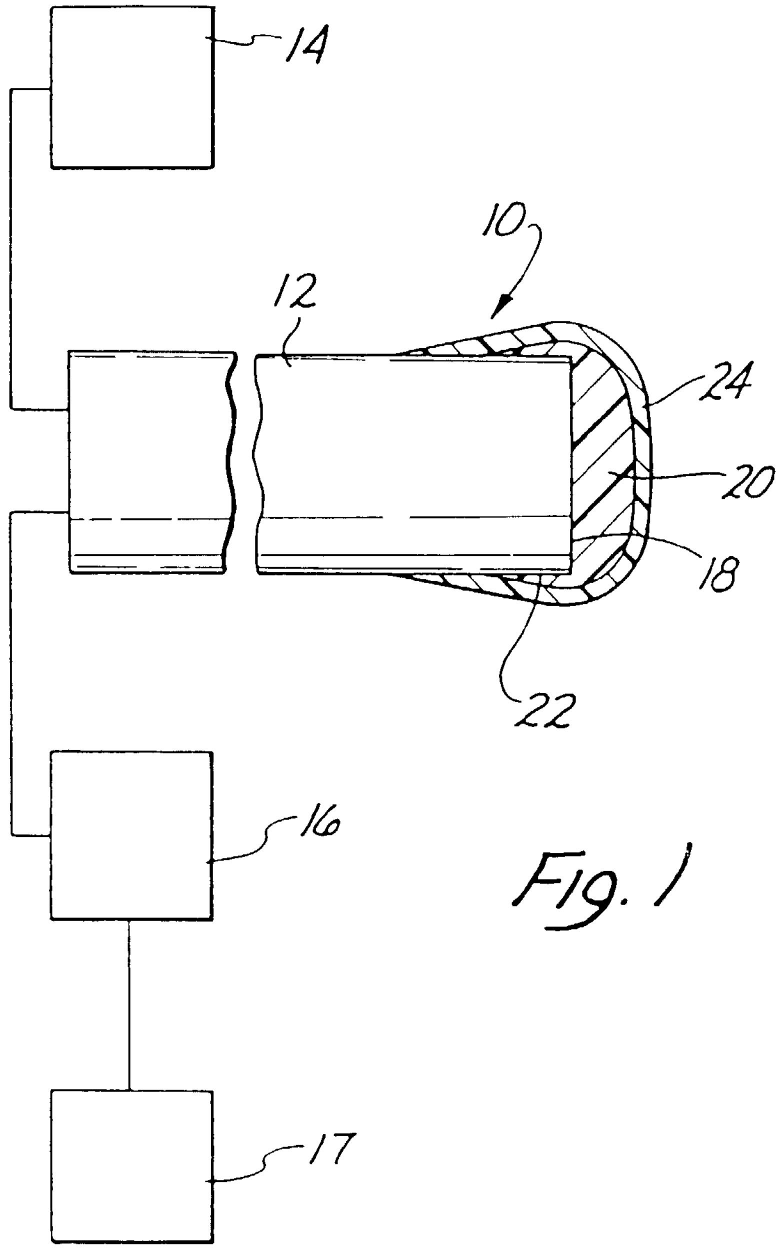


Fig. 1

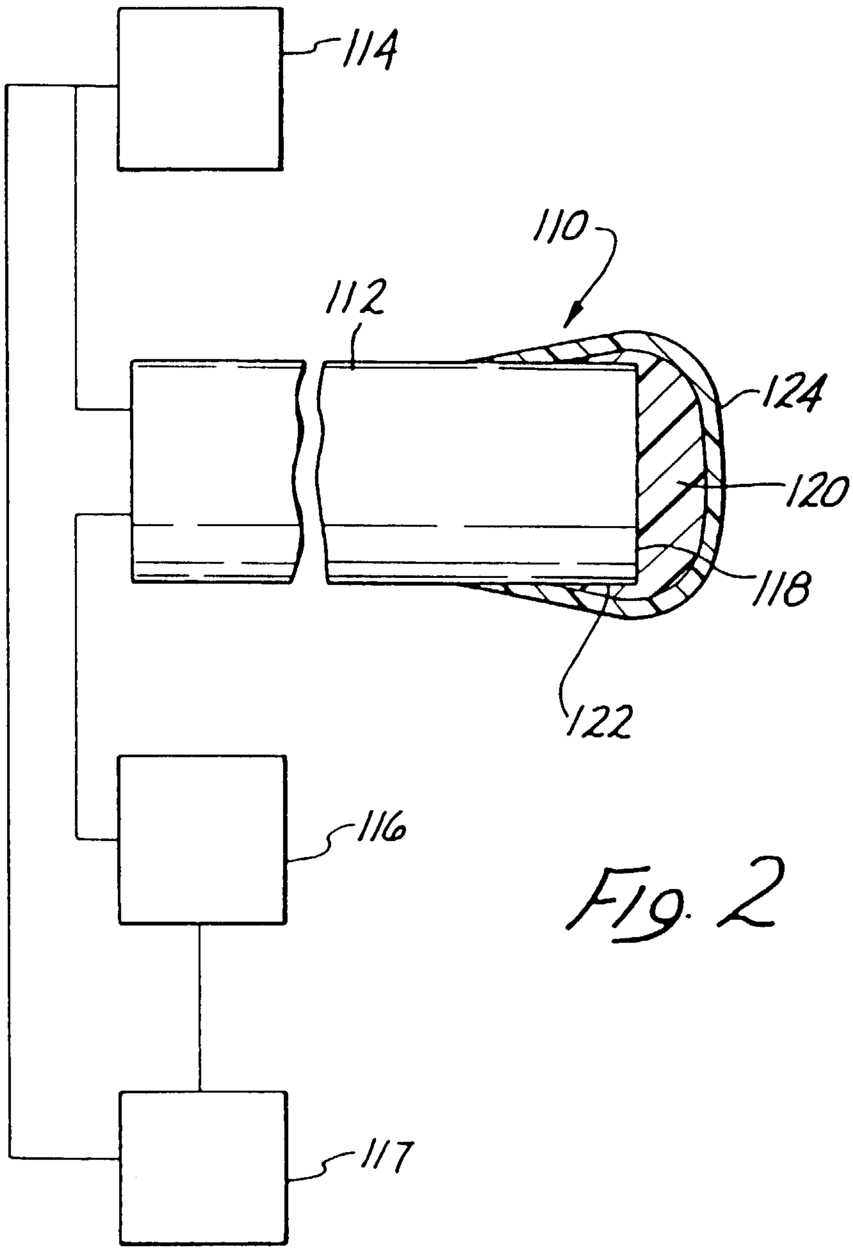


Fig. 2